# EUROPEAN PATENT APPLICATION

(11) **EP 4 703 469 A1**
(43) Date of publication of application: **04.03.2026**
(21) Application number: 25151251.3
(22) Date of filing: 10.01.2025
(51) Int. Cl.: C12N 9/22, A61K 40/11, A61K 48/00, C12N 15/90

(54) **FUSION PROTEINS, METHODS AND COMPOSITIONS FOR EFFICIENT SERINE INTEGRASE-MEDIATED GENE TRANSFER IN HUMAN CELLS**

(30) Priority: 26.08.2024 EP 24196550
(71) Applicant: Charité - Universitätsmedizin Berlin Körperschaft des öffentlichen Rechts, 10117 Berlin (DE)
(72) Inventor: WAGNER, Dimitrios Laurin, Houston, 77030 (US); KASSING, Isabell, 10589 Berlin (DE); IBRAHIM, Daniel, 10785 Berlin (DE); KATH, Jonas Christian, 10435 Berlin (DE)
(74) Representative: Doll, Markus Alexander

(57) **Abstract**

The present invention relates to the field of gene transfer, in particular, targeted gene transfer and enzymes for use in targeted gene transfer. In particular, the present invention provides a nucleic acid encoding a fusion protein having serine integrase activity, wherein the fusion protein comprises a serine integrase consisting of a protein having at least 80% sequence identity to the large serine integrase Pa01 and a nuclear localization sequence at the C-terminus of the serine integrase. The invention further provides a fusion protein encoded by said nucleic acid as well as methods of preparing an engineered cell using the nucleic acids and/or fusion proteins of the invention. Finally, the present invention provides various pharmaceutical compositions and their use in treating a disease or disorder.

## Description

The present invention relates to the field of gene transfer, in particular, targeted gene transfer, and enzymes for use in targeted gene transfer. In particular, the present invention provides a nucleic acid encoding a fusion protein having serine integrase activity, wherein the fusion protein comprises a serine integrase consisting of a protein having at least 80% sequence identity to the large serine integrase Pa01 and a nuclear localization sequence at the C-terminus of the serine integrase. The invention further provides a fusion protein encoded by said nucleic acid as well as methods of preparing an engineered cell using the nucleic acids and/or fusion proteins of the invention. Finally, the present invention provides various pharmaceutical compositions and their use in treating a disease or disorder.

Genome editing using designer nucleases, such as CRISPR-Cas, and their derivative technologies have been revolutionary tools to perform precise genetic interventions, such as inducing small insertions, deletions and base conversions. However, gene editing tools and conventional gene transfer with viral vectors are inefficient when transferring larger genetic information of more than 5 kb at precise locations.

Transposases, such as PiggyBac, have been successfully used to efficiently transfer large genetic cargo, but their random integration mode poses a high risk of malignant transformation through insertional mutagenesis and other forms of genotoxicity. For example, in a trial in Australia, 2/11 patients which received CD19-CAR T cells generated with a hyperactive version of PiggyBac, developed a CD4+ CAR+ T cell lymphoma and one patient succumbed to this gene-therapy-induced malignancy (Micklethwaite et al., 2021).

There is thus an unmet need of highly efficient enzymes that allow the safe and precise integration of large genetic cargo. Such tools could be used to develop "smart" cell therapies including multiple tumor-specific antigen receptors, performance and safety enhancing transgenes, as well as tunable synthetic circuits to regulate transgene expression. Further, many monogenetic disorders would benefit from large gene transfer methods: in patient cells with large deletions, these tools could be used to repair the part of their genome. In diseases caused by many diverse mutations in a gene with a large cDNA, tools with larger cargo capacity could be used to develop universal repair strategies suitable for all/most mutations.

Large serine integrases (LSR) are enzymes derived from bacteriophages, which are viruses that infect bacteria. These integrases mediate site-specific and unidirectional recombination, allowing for the precise insertion or excision of DNA segments at specific sequences (so called attachment sites or "landing pads") within the genome. This characteristic makes them powerful tools for gene therapy, because they can integrate very large pieces of DNA of more than 5,000 bp and at least up to 100,000 bp into precise locations. To date, only a few large serine integrases, namely PhiC31 and Bxb1, have been evaluated for their utility to insert genes in primary human T cells. A recent study (Durrant et al., 2023) identified additional promising serine integrases for use in mammalian cells through metagenomic mining, including serine integrases Pa01 and Kp03. While Kp03 displayed significant off-target integrations, presumably due to pseudosites in the genome of humans, Pa01 was characterized by a high efficiency and specificity as only very few off-target events were detected in cancer cell lines.

In light of this, the inventors addressed the problem of providing large serine integrase variants with improved potency for gene transfer in human cells, in particular primary human cells. This problem is solved by the present invention, in particular, as laid out in the appended claims.

In particular, the present invention provides, in a first aspect, a nucleic acid encoding a fusion protein comprising
a) a large serine integrase consisting of a protein having at least 80% sequence identity to SEQ ID NO: 1 and
b) a nuclear localization signal (NLS) at the C-terminus of the serine integrase.

Accordingly, the present invention also provides a fusion protein comprising a large serine integrase having at least 80% sequence identity to SEQ ID NO: 1 and a NLS at the C-terminus of the serine integrase encoded by said nucleic acid. The term "protein" as used herein includes proteins, polypeptides, and peptides.

Large serine integrases form a group of site-specific DNA recombinases that are derived from bacteriophages and mediate phage integration into a host genome. Like other site-specific DNA recombinases, large serine integrases catalyse breaking and rejoining of DNA strands at specific points, thereby bringing about precise genetic rearrangements that can be used to target-specifically integrate large DNA fragments into a recipient DNA. The exact integration mechanism, i.e,, the recombination mechanism catalysed by large serine integrases relies on a specific attachment site in the host chromosome known as the attB site (attachment site in Bacteria) as well as a corresponding phage attachment site on the circular phage genome known as attP site (attachment site in Phage). Both the attP and the attB sites comprise binding sites for the large serine integrase and are characterized by a characteristic central dinucleotide core. Following binding of the att sites by the integrase, integrase dimers bound to attP and attB interact to form a synaptic complex. The large serine integrase then promotes double-strand cleavage at the dinucleotide cores of both sites so that each DNA 'half-site' is covalently attached to an integrase subunit via a phosphodiester linking the active site serine residue to the recessed 5' DNA end. The cleaved half-sites along with the attached integrase subunits then swap positions by a rotational movement, and the DNA ends are religated in this recombinant configuration to obtain an integrated prophage flanked by the recombinant sites *attL* (attachment site on the left) and *attR* (attachment site on the right) (Stark, 2017). The integration mechanism mediated by large serine integrases does not require any additional host-encoded proteins.

In the context of the invention, a large serine integrase or a "protein with large serine integrase activity" can therefore be defined as an enzyme capable of integrating a DNA cargo sequence into a target region of a host DNA by binding to specific attachment sites (att) within both a nucleic acid comprising the DNA cargo sequence and within the host DNA, introducing double-strand breaks into the nucleic acid comprising the DNA cargo sequence and the host DNA and mediating strand exchanges by a rotational mechanism, thereby generating genetic rearrangements. For the sake of convenience, the terms "large serine integrase" and "serine integrase" are used interchangeably herein. Thus, whenever a "serine integrase" or a "protein having serine integrase activity" is mentioned herein, it should be understood that these terms are meant to be synonymous with "large serine integrase" and "protein with large serine integrase activity", respectively.

The fusion protein according to the invention comprises a protein exhibiting large serine integrase enzymatic activity, wherein the at least 80% amino acid identity to SEQ ID NO: 1, optionally, at least 85% sequence identity, at least 90% sequence identity, at least 95% sequence identity, at least 96% sequence identity, at least 97% sequence identity, at least 98% sequence identity or at least 99% sequence identity. Optionally, the protein exhibiting large serine integrase enzymatic activity comprises only one, two or three amino acid differences to SEQ ID NO: 1. In some embodiments, the amino acid sequence of the protein exhibiting large serine integrase enzymatic activity may also lack 1 to 10 C-terminal amino acids of SEQ ID NO: 1.

The fusion protein according to the invention may also comprise a large serine integrase with an amino acid sequence having 100% sequence identity to SEQ ID NO: 1. Such a large serine integrase is known under the name Pa01 and has been described in WO2023081762 and Durrant et al., 2022. It may be encoded by a nucleic acid having a sequence of SEQ ID NO: 1. Pa01 was found to be highly specific with no detected off-target events in cancer cell lines.

Importantly, fusing an NLS to the C-terminus of the serine integrase having at least 80% sequence identity to SEQ I D NO: 1 does not impair or abrogate the enzymatic activity of the serine integrase. Quite to the contrary, the inventors surprisingly found that the ability of Pa01 to insert DNA into the genome of cells, in particular primary human cells such as, e.g., primary human T cells, can be further increased by fusing the NLS to the C-terminal end of Pa01. In contrast, fusing a NLS to the N-terminal end of Pa01 significantly decreased its efficacy. Likewise, fusing NLS to both the C- and N-terminal ends also yielded reduced integration rates.

Thus, the fusion protein of the invention is characterized by a serine integrase activity that is at least 10%, at least 20%, at least 30%, at least 40%, at least 50%, at least 60%, at least 70%, at least 80%, at least 90%, at least 100%, at least 110%, at least 120%, at least 130%, at least 140%, at least 150%, at least 160%, at least 170%, at least 180%, at least 190% or at least 200% higher than the serine integrase activity of Pa01 that does not comprise a C-terminal NLS.

Nuclear localization signals (also referred to as nuclear localization sequence or NLS) are short peptide sequences that label a protein for import into the cell nucleus by nuclear transport through nuclear pore complexes. These signals are recognized by importin proteins, which mediate the transport of cargo proteins across the nuclear envelope. Currently known NLS can be divided into different types and categories.

So-called "classical" NLS (cNLS) encompass two distinct categories termed "monopartite" (MP) and "bipartite" (BP). The former are single clusters formed of 4-8 basic amino acids typically containing 4 or more positively charged lysines or arginines. MP NLS are usually characterized by the consensus sequence KX₁X₂X₃, wherein X₁ and X₃ can be either K or R and X₂ can be any amino acid (Lu et al., 2021). Representative examples of an MP NLS comprising the consensus sequence KX₁X₂X₃ that can be used in the context of the present invention are the NLS of SV40 large T-antigen having a sequence of PKKKRKV (SEQ ID NO: 2) or Myc (DYPAAKRVKLD, SEQ ID NO: 3). An additional classical monopartite NLS that may be used in the context of the invention is PAAKRVKLD (SEQ ID NO: 4).

BP NLS are characterized by two clusters of 2-3 positively charged amino acids that are separated by a 10-12 amino acid linker region comprising one or more P residues. BP NLS are thus characterized by the consensus sequence X₁(X₂)₁₀₋₁₂KRX₃K, where X₁ can be either K or R and X₂ and X₃ can be any amino acid. The upstream and downstream clusters of amino acids are interdependent and indispensable, and jointly determine the localization of the protein in the cell. Exemplary BP NLS that can be used in the context of the invention include, e.g., the NLS of nucleoplasmin (KRPAATKKAGQAKKKK, SEQ ID NO: 5), 53BP1 (GKRKLITSEEERSPAKRGRKS, SEQ ID NO: 6), ING4 (KGKKGRTQKEKKAARARSKGKN, SEQ ID NO: 7), IER5 (RKRCAAGVGGGPAGCPAPGSTPLKKPRR, SEQ ID NO: 8) or ERK5 (RKPVTAQERQREREEKRRRRQERAKEREKRRQERER, SEQ ID NO: 9) (Lu et al. 2021).

In addition to the "classical" NLS described above, a variety of "non-classical" NLS (ncNLS) exist that are neither similar to the canonical signals nor rich in arginine or lysine residues. Well known examples for such ncNLS are ncNLS of the "proline-tyrosine" type commonly referred to as PY-NLS. These PY-NLS are characterized by 20-30 amino acids that assume a disordered structure, consisting of N-terminal hydrophobic or basic motifs and C-terminal X₁(X₂)₂₋₅PY motifs, wherein X₁ can be any of R, K or H and (X₂)₂₋₅ can be any amino acid sequence of 2-5 residues. Exemplary PY-NLS known to the skilled person that may be used in the context of the present invention include the NLS of human heterogeneous nuclear ribonucleoprotein A1 (FGNYNNQSSNFGPMKGGNFGGRSSGPY, SEQ ID NO: 10) or of the human cytomegalovirus protein UL79 (TLLLRETMNNLGVSDHAVLSRKTPQPY, SEQ ID NO: 11) (Lu et al., 2021). Other ncNLS that may be used in the context of the present invention have amino acid sequences of, e.g., KRTADGSEFES (SEQ ID NO: 12), YRPRPKRTCVI (SEQ ID NO: 13), or SRRRKANPTKLSENAKKLAKEVEN (SEQ ID NO: 14).

Thus, in a preferred embodiment of the invention, the NLS at the C-terminus of the serine integrase is a classical "monopartite" NLS comprising the consensus motif KX₁X₂X₃. Preferably, the NLS is an NLS of SV40 large T-antigen having an amino acid sequence of SEQ ID NO: 2. Alternatively, the classical "monopartite" NLS may have an amino acid sequence of SEQ ID NO: 3 or 4. Optionally, multiple of the herein disclosed monopartite NLS may also be combined for increased nuclear import and improved recombination activity in cells, in particular primary human cells. Thus, the NLS of the fusion protein of the invention may comprise or consist of combinations of two or more of the herein disclosed monopartite NLS, e.g. a combination of SEQ ID NO: 2 and 3, SEQ ID NO: 2 and 4, SEQ ID NO: 3 and 4 or SEQ ID NO: 2, SEQ ID NO: 3 and SEQ ID NO: 4. Alternatively or in addition, the NLS of the fusion protein of the invention may also comprise or consist of multiple copies of any of the herein disclosed monopartite NLS, e.g. it may comprise or consist of at least two copies of SEQ ID NO: 2, SEQ ID NO: 3 and/or SEQ ID NO: 4.

In another embodiment, the NLS at the C-terminus of the serine integrase is a classical "bipartite" NLS comprising the consensus sequence X₁(X₂)₁₀₋₁₂KRX₃K. The NLS may, e.g. be the BP NLS of nucleoplasmin having an amino acid sequence of SEQ ID NO: 5 or may be an NLS of any of SEQ ID NO: 6-9. Optionally, multiple of the herein disclosed bipartite NLS may also be combined with each other.

Thus, the NLS of the fusion protein of the invention may comprise or consist of combinations of two or more of the herein disclosed bipartite NLS, e.g. a combination of SEQ ID NO: 5 and 6, SEQ ID NO: 5 and 7, SEQ ID NO: 5 and 8, SEQ ID NO: 5 and 9, SEQ ID NO: 6 and 7, SEQ ID NO: 6 and 8 , SEQ ID NO: 6 and 9, SEQ ID NO: 7 and 8, SEQ ID NO: 7 and 9 or SEQ ID NO: 8 and 9. It may also comprise combinations of more than two of the herein disclosed bipartite NLS, e.g., of three, four, or all of the bipartite NLS according to SEQ ID NO: 5-9. Alternatively or in addition, the NLS of the fusion protein of the invention may also comprise or consist of multiple copies of any of the herein disclosed bipartite NLS, e.g. it may comprise or consist of at least two copies of SEQ: 5, SEQ ID NO: 6, SEQ ID NO: 7, SEQ ID NO: 8 and/or SEQ ID NO: 9. For instance, in one particularly preferred embodiment of the invention, the NLS at the C-terminus of the serine integrase comprises two copies of the NLS of nucleoplasmin having an amino acid sequence of SEQ ID NO: 5. Accordingly, the NLS at the C-terminus of the serine integrase may have an amino acid sequence of KRPAATKKAGQAKKKKGSKRPAATKKAGQAKKKK (SEQ ID NO: 15). In some embodiments, any of the herein disclosed monopartite NLS of SEQ ID NOs: 2-4 may also be combined with any of the herein disclosed bipartite NLS of SEQ ID NOs: 5-9.

The NLS of the fusion protein according to the invention may also be a non-classical NLS such as, e.g., a PY-NLS of, e.g., SEQ ID NO: 10 or 11 as disclosed herein. The NLS may also have an amino acid sequence of SEQ ID NO: 12, 13 or 14. Optionally, non-classical NLS may also be combined with each other. Thus, the NLS of the fusion protein of the invention may comprise or consist of combinations of two or more of the herein disclosed non-classical NLS, e.g. a combination of SEQ ID NO: 10 and 11, SEQ ID NO: 10 and 12, SEQ ID NO: 10 and 13, SEQ ID NO: 10 and 14, SEQ ID NO: 11 and 12, SEQ ID NO: 11 and 13, SEQ ID NO: 11 and 14, SEQ ID NO: 12 and 13, SEQ ID NO: 12 and 14, or SEQ ID NO: 13 and 14. It may also comprise combinations of more than two of the herein disclosed non-classical NLS, e.g., of three, four, or all of the non-classical NLS according to SEQ ID NO: 10-14. Alternatively or in addition, the NLS of the fusion protein of the invention may also comprise or consist of multiple copies of any of the herein disclosed non-classical NLS, e.g. it may comprise or consist of at least two copies of SEQ: 10, SEQ ID NO: 11, SEQ ID NO: 12, SEQ ID NO: 13 and/or SEQ ID NO: 14. Ins some embodiments, any of the herein disclosed monopartite NLS of SEQ ID NOs: 2-4 may also be combined with any of the herein disclosed bipartite NLS of SEQ ID NOs: 5-9 and/or any of the herein disclosed non-classical NLS of SEQ ID NO: 10-14.

Optionally, the NLS may be linked to the C-terminus of the serine integrase via a suitable spacer sequence. The spacer sequence should be chosen so that it does not interfere with the proper folding of the final fusion protein into a functional serine integrase, while ensuring that the NLS is displayed on the surface of the fusion protein. It may, e.g., be 1 to 50 amino acids long. A person skilled in the art will be able to identify suitable spacer sequences. However, preferably, the NLS is directly fused to the C-terminus of the serine integrase, i.e., without the need of a spacer sequence.

In some embodiments, the fusion protein comprises at least one other segment at the C-terminus or between the serine integrase and the NLS.

Said at least one other segment may comprise, e.g., a tag allowing for protein purification and/or detection. Suitable tags known to the skilled person that can be used for this purpose include but are not limited to an HA-tag, FLAG-tag, His-tag, Myc-tag, ALFA-tag, polyglutamate tag, polyarginine tag, Fc-tag or a fluorescent protein such as GFP or mCherry. Optionally, the tag may be removably attached to the C- terminus of the fusion protein, e.g., via a suitable cleavable linker sequence such as, e.g., a chemically labile linker or an enzyme-cleavable linker. The skilled person will be aware of suitable linkers from the state of the art.

In a further embodiment, the at least one other segment at the C-terminus of the fusion protein or between the serine integrase and the NLS may comprise one or more additional enzymes. For instance, the fusion protein according to the invention may comprise a large serine integrase consisting of a protein having at least 80% sequence identity to SEQ ID NO: 1 and, fused to the C-terminus of said serine integrase, a gene editing enzyme, e.g. a prime editor consisting of CRISPR-Cas9 nickase and a reverse transcriptase (RT) and the NLS. Such a fusion protein may e.g. resemble a genome editing tool called PASTE ("programmable addition via site-specific targeting elements") as disclosed in WO 2022087235 A1 and Yarnall et al., 2023, in which a Bxb1 integrase is fused with a nickase and a reverse transcriptase. In such an embodiment, any of the NLS, the CRISPR-Cas9 nickase or the reverse transcriptase may form the C-terminus of the fusion protein. However, in a preferred embodiment, the fusion protein comprises the serine integrase consisting of a protein having at least 80% sequence identity to SEQ ID NO: 1 with its C-terminus fused to the reverse transcriptase, wherein the C-terminus of the reverse transcriptase is fused to the CRISPR-Cas9 nickase and wherein the NLS is fused to the C-terminus of the CRISPR-Cas9 nickase. Alternatively, the fusion protein comprises the serine integrase consisting of a protein having at least 80% sequence identity to SEQ ID NO: 1 with its C-terminus fused first to the NLS, wherein the C-terminus of the NLS is in turn fused to the reverse transcriptase, and wherein the C-terminus of the reverse transcriptase is fused to the CRISPR-Cas9 nickase.

Surprisingly, the inventors found that fusing a NLS to the N-terminal end of the Pa01 serine integrase significantly decreased its efficacy which contrasts data from other serine integrases, like Bxb1 and PhiC31, which have been modified with NLS at the N-Terminus without disrupting its recombination activity. Therefore, preferably, the N-terminus of the fusion protein encoded by the nucleic acid of the invention is not modified with an NLS. Accordingly, the N-terminus of the serine integrase preferably is the N-terminus of the fusion protein encoded by the nucleic acid of the invention. However, in some embodiments, the N-terminus of the serine integrase may be fused, e.g., to a tag allowing for protein purification and/or detection, as described herein. In such an embodiment, the tag is preferably removably attached to the N-terminus of the serine integrase, e.g., via a suitable cleavable linker sequence as defined herein. If the N-terminus of the serine integrase is fused, e.g., to a tag, the C-terminus of the fusion protein according to the invention may not comprise a tag and vice versa. However, in some embodiments, it may also be possible that both the N-terminus and the C-terminus of the serine integrase comprises, e.g., a tag.

An exemplary fusion protein of the present invention as used in the Examples below comprises a serine integrase of SEQ ID NO: 1 and an NLS of SEQ ID NO: 2 and thus may have an amino acid sequence of SEQ ID NO: 16. It may be encoded by a nucleic acid comprising a nucleic acid sequence according to SEQ ID NO: 17. An alternative fusion protein of the present invention comprises a serine integrase of SEQ ID NO: 1 and an NLS of SEQ ID NO: 4 and thus may have an amino acid sequence of SEQ ID NO: 18. It may be encoded by a nucleic acid comprising a nucleic acid sequence according to SEQ ID NO: 19. A further exemplary fusion protein of the present invention comprises a serine integrase of SEQ ID NO: 1 and an NLS of SEQ ID NO: 14 and thus may have an amino acid sequence of SEQ ID NO: 20. It may be encoded by a nucleic acid comprising a nucleic acid sequence according to SEQ ID NO: 21. A further exemplary fusion protein of the present invention comprises a serine integrase of SEQ ID NO: 1 and an NLS of SEQ ID NO: 5 and thus may have an amino acid sequence of SEQ ID NO: 22. It may be encoded by a nucleic acid comprising a nucleic acid sequence according to SEQ ID NO: 23. A further exemplary fusion protein of the present invention comprises a serine integrase of SEQ ID NO: 1 and an NLS comprising two copies of SEQ ID NO: 5. It may thus have an amino acid sequence of SEQ ID NO: 24. It may be encoded by a nucleic acid comprising a nucleic acid sequence according to SEQ ID NO: 25.

In the context of the invention, a "nucleic acid" is a polymeric compound comprised of covalently linked subunits called nucleotides. In a preferred embodiment, the nucleic acid according to the invention encoding the herein disclosed fusion protein is RNA, which may be double-stranded or single-stranded. The RNA preferably is a messenger RNA (mRNA), e.g. an *in vitro* transcribed mRNA, typically in a form comprising modified nucleotides and/or structures that makes the RNA more stable and less immunogenic than classical RNA. For instance, the mRNA may be a nucleoside-modified mRNA, i.e., a synthetic RNA comprising one or more naturally modified nucleosides or synthetic nucleoside analogues, e.g., the mRNA according to the invention may comprise one or more modified nucleosides selected from the group comprising 5-methylcytidine, N4-acetylcytidine, Pseudouridine, N1-methylpseudouridine, 6-methoxyuridine, 2-thiouridine, N6-methyladenosine, N1-methyladenosine, N6,2'-O-dimethyladenosine or 8-Oxo-7,8-dihydroguanosine. The *in vitro* preparation of mRNA is carried out according to standard procedures known to the skilled person from the prior art. For example, mRNAs can be generated by chemical synthesis, which is typically done by commercial companies on a customized basis. Alternative methods for generating mRNA molecules are well known to the skilled person and include, e.g., *in vitro*-transcription or *in vivo* transcription from DNA templates introduced into cells.

The nucleic acid may also be a viral vector based on RNA, e.g., a retroviral vector such as a lentiviral vector encoding the fusion protein of the invention. It may also be a circular RNA, e.g., a circular single-stranded or double-stranded RNA, that is, optionally, provided in a virus-like particle.

In an alternative embodiment, the nucleic acid of the invention may also be DNA. The DNA may be double-stranded or single-stranded DNA. It can be, e.g., cDNA, genomic DNA, synthetic DNA, and semi-synthetic DNA. The DNA may also be an expression cassette in a DNA vector. The DNA expression cassette thus encodes the fusion protein of the invention, i.e., the vector may be, e.g., a vector that, when being expressed, results in the generation of the fusion protein according to the invention. Examples of such expression vectors include but are not limited to minicircles, plasmids, cosmids, phages, viruses or artificial chromosomes.

Thus, the present invention also provides an expression vector comprising the nucleic acid of the present invention. The expression vector may be made of RNA or DNA as disclosed herein.

The expression vector preferably further comprises at least a regulatory region of a gene. The regulatory region may be a transcriptional regulatory region, e.g., selected from the group consisting of a promoter, an enhancer, a silencer, a locus control region, and a border element, typically, at least a promotor. Promoters and/or other expression control regions can be operably linked with the nucleic acid encoding the inventive fusion protein or to a nucleotide sequence which is complementary to or which hybridizes to said nucleotide sequence, e.g., to regulate expression of the fusion protein in a quantitative or in a tissue-specific manner. The promotor can be a native or, preferably, a heterologous promoter. The selection of promoters includes, e.g., strong, weak, inducible, tissue-specific and developmental-specific promoters. The promoter can be a non-viral promoter or a viral promoter. The inventive recombinant expression vectors can be designed for either transient expression, for stable expression, or for both. Also, the recombinant expression vectors can be made for constitutive expression or for inducible expression. Furthermore, the vector comprising the nucleic acid of the invention may include one or more marker genes, which allows for selection or detection (e.g. via a marker gene expressing GFP) of transduced or transfected hosts.

The nucleic acid of the invention is preferably further codon-optimized for expression in a target organism and/or the target tissue. For this, isolated triplet codons of the nucleotide sequence encoding the fusion protein are replaced by synonymous codons in the course of *in vitro* mutagenesis in order to adapt the expression rate of the fusion protein to the preferred codon usage of the target organism or the target tissue. Codon optimization leads to an improved expression of the encoded polypeptide in a selected host organism, e.g., in a human cell. Tables for appropriately adjusting a nucleic acid sequence to the host cell's specific transcription/translation machinery are known to a skilled person. In general, it is preferred to adapt the G/C-content of the nucleotide sequence to the specific host cell conditions. Preparation and purification of such nucleic acids and/or derivatives are usually carried out by standard procedures.

As large serine integrases naturally evolved to deliver long stretches of a viral genetic code, there is no reported upper limit on the size of the donor DNA, with reports demonstrating successful integration of up to 43 kb into mammalian cells (Hosur et al., 2022). Thus, the herein disclosed fusion protein of the invention is capable of site-specifically delivering exceptionally large cargo sequences of at least up to 1000 bp, at least up to 2000 bp, at least up to 5000 bp, at least up to 8000 bp, at least up to 10000 bp, at least up to 20000 bp, at least up to 30000 bp, of at least up to 40000 bp, up to 50000 bp, up to 75000 bp and possibly even up to 100000 bp at a remarkable efficiency. Thus, the fusion protein according to the invention is excellently suited for modifying the genome of cells, such as mammalian, e.g., human cells. It therefore represents a valuable tool for various biotechnological applications, e.g. in the production of therapeutically effective cells, in cell line engineering or the production of protein expression cell lines.

In a further aspect, the present invention thus also provides a method of preparing an engineered cell comprising a targeted insertion of a cargo sequence in its genomic DNA.

The method comprises steps of:
a. optionally, inserting, site-specifically, one or more genomic attachment sites comprising a nucleic acid sequence having at least 90% sequence identity to any of SEQ ID NO: 31 or SEQ ID: 51 into the genomic DNA of a cell,
b. contacting the cell with a nucleic acid, e.g., an mRNA, encoding a protein with serine integrase activity comprising an amino acid sequence having at least 80% sequence identity to SEQ ID NO: 1 or with a protein with serine integrase activity comprising an amino acid sequence having at least 80% sequence identity to SEQ ID NO: 1 and with a DNA comprising i) one or more vector attachment sites comprising a nucleic acid sequence having at least 90% sequence identity to any of SEQ ID NO: 31 or SEQ ID NO: 51 and ii) the cargo sequence,
   wherein the one or more genomic attachment sites and the one or more vector attachment sites correspond to each other to enable serine integrase-mediated recombination.

In the context of the invention, the protein with serine integrase activity comprising an amino acid sequence having at least 80% sequence identity to SEQ ID NO: 1 may be, e.g., a wild type Pa01 serine integrase having an amino acid sequence of SEQ ID NO: 1. Accordingly, the nucleic acid encoding such a protein may have a nucleic acid sequence of SEQ ID NO: 74. However, the protein with serine integrase activity comprising an amino acid sequence having at least 80% sequence identity to SEQ ID NO: 1 may also be a modified Pa01 serine integrase, e.g., it may be a fusion protein in which a Pa01 serine integrase has been fused to another segment, e.g., a protein tag, another enzyme or an NLS.

However, preferably, the nucleic acid encoding a protein with serine integrase activity comprising an amino acid sequence having at least 80% sequence identity to SEQ ID NO: 1 is a nucleic acid of the present invention. Likewise, the protein with serine integrase activity comprising an amino acid sequence having at least 80% sequence identity to SEQ ID NO: 1 is preferably a fusion protein according to the present invention.

The method of the invention may be an *in vivo*, *ex vivo* or *in vitro* method. In an *in vitro* or ex *vivo* embodiment, it can further, optionally, comprise a step c. of selecting cells in which the cargo sequence has been inserted. In one embodiment, the method of the invention further comprises a step d. of formulating the cell of step b. or c. as a pharmaceutical composition, optionally, including packaging.

Accordingly, the present invention also discloses the use of a nucleic acid or the fusion protein of the present invention for preparing an engineered cell comprising a cargo sequence in its genomic DNA.

The cell can be of any species, e.g., a mammalian cell, a yeast cell, a bacterial cell, a plant cell, a fish cell, an avian cell or an insect cell. Preferably, the cell is a mammalian cell, such as a mouse, hamster, rat or human cell, most preferably, a human cell.

The cell can be a cultured cell or a primary cell, i.e., isolated directly from an organism, e.g., a human. Preferably, the cell is a primary human cell. The cell can be an adherent cell or a suspension cell, i.e., a cell that grows in suspension. Because large serine integrases integrase can work efficiently in both resting and dividing cell types, the cell can be a non-dividing cell, such as, e.g., a post-mitotic brain, muscle or kidney cell. It may however also be a stem cell. The cell can e.g., be a liver cell or a lung cell.

While the cell can be of any cell type, can originate from any type of tissue, and can be of any developmental stage, in some embodiments, the cell preferably is a peripheral blood leukocyte (PBL) or a peripheral blood mononuclear cell (PBMC). More preferably, the cell is a T cell or T cell precursor, in particular, a human T cell. The T cell can be any T cell, such as a cultured T cell, e.g. a primary T cell, or a T cell from a cultured T cell line, or a T cell obtained from a mammal. Preferably, the cell is a T cell or T cell precursor from a human patient, in particular, from a human patient who is to be treated. The T cell, of autologous or allogeneic origin, can be obtained from numerous sources, such as blood, bone marrow, lymph nodes, thymus, spleen, or other tissues or body fluids. The T cell may also be derived from an induced pluripotent stem cell (iPSC). T cells can also be enriched for or purified. Preferably, the T cell is a human T cell. More preferably, the T cell is a T cell isolated from a human, e.g., a human patient. The T cell can be any type of T cell, e.g., a CD8+ cell or a CD4+ cell. It can be an alpha beta T cell or a gamma delta T cell. It can be of any developmental stage, including but not limited to tumor infiltrating cells (TILs), effector cells, central effector cells, memory T cells, naive T cells, or regulatory T cells. Advantageously, the inventors have found that the protein of the invention is highly active and non-toxic in human primary T cells.

The DNA comprising the cargo sequence can for example be provided as double-stranded DNA, which is herein also referred to as an integration vector or donor plasmid. It can be a circular DNA e.g., a minicircle, nanoplasmid or microcircle. It may however also be a linear DNA. Preferably, it is a circular DNA. Optionally, the cargo sequence comprises an open reading frame operably linked to a promotor, wherein the open reading frame may encode one or more genes of interest, e.g., one or more tumor-specific antigen receptors, e.g., a chimeric antigen receptor (CAR) or a TCR. Alternatively or additionally, the cargo sequence may also comprise sequences encoding at least one miRNA or shRNA. The open reading frame may alternatively or additionally encode a marker, e.g. a drug resistance gene, an enzyme, a growth factor, a natural and/or synthetic receptor or transcription factor, or a fluorescent protein.

To be integrated into the genome of the cell with the aid of a protein with serine integrase activity comprising an amino acid sequence having at least 80% sequence identity to SEQ ID NO: 1, such as, e.g., the fusion protein according to the invention, the nucleic acid comprising the cargo sequence, i.e., the integration vector, must further comprise at least one attachment (att) site that can be recognized and bound by the serine integrase, e.g., the fusion protein according to the present invention. Because this attachment site is provided on the integration vector, it is referred to in the context of the present invention as vector attachment site or simply as attV site (attachment site in Vector). Thus, in the context of the present invention, the term "vector attachment site" refers to a nucleic acid sequence in the nucleic acid comprising the cargo sequence, i.e., the integration vector, that can be recognized and bound by a protein with serine integrase activity comprising an amino acid sequence having at least 80% sequence identity to SEQ ID NO: 1 to enable serine integrase-mediated recombination.

The attV site may be derived either from the naturally occurring (native) attP or the native attB site of the Pa01 serine integrase. In the context of the present invention, a sequence is considered to be "derived from" the attP or attB site of the Pa01 serine integrase if it is i) identical to the native attB or attP site (i.e., identical to SEQ ID NO: 26 or 30, respectively), ii) a shortened version of the native attP or attB site of the Pa01 serine integrase and/or iii) if at least one of the two nucleotides TC that form the central dinucleotide core of the native attP or attB sites of the Pa01 serine integrase have been substituted by a different nucleotide.

Thus, in one embodiment, the attV site in the DNA comprising the cargo sequence, i.e., the integration vector, may be identical or nearly identical to the naturally occurring, unmodified attB site of Pa01 and may thus have a nucleic acid sequence having at least 90% sequence identity to
CCGGTTTCCCTTCGCACCCGCACCGCGGCTTCGAGACCGTGACCTACATGCTCGAAGGGC GTATGCGCCACGAAGACCACCTCGGCAATCGCGGCCTGCTCAAG (SEQ ID NO: 26). It has been found that the attB site of Pa01 can be shortened to a certain extent without causing a loss of the integration efficiency mediated by the Pa01 serine integrase. Thus, in some embodiments, the attV site may also comprise or consist of a shortened Pa01 attB site with a nucleic acid sequence having at least 90% sequence identity to GCACCGCGGCTTCGAGACCGTGACCTACATGCTCGAAGGGCGTATGCGCCACGAAGACC ACCTCG (SEQ ID NO: 27). In fact, the attV may also comprise or consist of a minimal Pa01 attB site consisting of only 40 bp with a nucleic acid sequence having at least 90% sequence identity to GACCGTGACCTACATGCTCGAAGGGCGTATGCGCCACGAA (SEQ ID NO: 28). It may also comprise or consist of a 33 bp-minimum attB sequence with a nucleic acid sequence having at least 90% sequence identity to CCGTGACCTACATGCTCGAAGGGCGTATGCGCC (SEQ ID NO: 29) first reported in Durrant et al., 2022. Both minimal Pa01 attB sites of SEQ ID NO: 28 and 29 were demonstrated to enable integration efficiencies comparable to that of the unmodified full-length attB site of Pa01. In preferred embodiments, the attV may comprise or consist of a nucleic acid sequence having at least 95% or at least 99% sequence identity to any of SEQ ID NO: 26-29. In some embodiments, the attV may also comprise or consist of a nucleic acid sequence having 100% sequence identity to any of SEQ ID NO: 26-29.

In an alternative embodiment, the attV site in the integration vector may also be derived from the naturally occurring unmodified attP site of the Pa01 serine integrase. It may thus comprise or consist of a nucleic acid sequence having at least 90% sequence identity to GTAACGCTCTTCGAGAAAGCAGATTCTCATATCCATCTTGAGTCTTCTTTCTCGCAAGACAA CACGAAATAGACACAGTCTCTTCCCTAGCTGTACACTGAGCC (SEQ ID NO: 30). Also the attP site of Pa01 can be shortened to a certain extent without causing a loss of the integration efficiency mediated by the serine integrase, Thus in some embodiments, the attV site may also comprise or consist of a shortened Pa01 attP site having a nucleic acid sequence that has 90% sequence identity to CGAGAAAGCAGATTCTCATATCCATCTTGAGTCTTCTTTCTCGCAAGACAACACGAAATAGA CACAGTCTCTTCCCTAGCTG (SEQ ID NO: 69) or to TCATATCCATCTTGAGTCTTCTTTCTCGCAAGACAACACGAAATAGACACAG (SEQ ID NO: 70). The attV may also comprise or consist of a minimal Pa01 attP site consisting of only 36 bp with a nucleic acid sequence having at least 90% sequence identity to ATCTTGAGTCTTCTTTCTCGCAAGACAACACGAAAT (SEQ ID NO: 71). In preferred embodiments, the attV may comprise or consist of a nucleic acid sequence having at least 95% or at least 99% sequence identity to any of SEQ ID NO: 30, 69, 70 or 71. In some embodiments, the attV may also comprise or consist of a nucleic acid sequence having 100% sequence identity to any of SEQ ID NO: 30, 69, 70 or 71.

Since the fusion protein according to the invention comprises a serine integrase having at least 80% sequence identity to Pa01 (SEQ ID NO: 1), it can recognize and bind to an attachment site comprising or consisting of a nucleic acid sequence having at least 90% sequence identity to any of SEQ ID NO: 26, 27, 28, 29, 30, 69, 70 or 71.

In the context of the invention, "contacting" a cell with a nucleic acid encoding a protein with serine integrase activity comprising an amino acid sequence having at least 80% sequence identity to SEQ ID NO: 1 (e.g., a nucleic acid of the present invention) or with a protein with serine integrase activity comprising an amino acid sequence having at least 80% sequence identity to SEQ ID NO: 1 (e.g., a fusion protein of the invention), and with the integration vector comprising the cargo sequence means that the nucleic acid or the protein, together with the integration vector, are delivered into the cell by any suitable means known to the skilled person. "Contacting" thus occurs under conditions that allow the uptake of the nucleic acid or protein as well as the integration vector. The delivery of the nucleic acid or protein and the integration vector into the cell may comprise physical transfection methods such as electroporation, microinjection or biolistic particle delivery as well as chemical methods such as calcium phosphate-based transfection, lipofection, or the use of dendrimers or cationic polymers such as DEAE-dextran or polyethyllenimine. Nucleic acids may also be introduced into the cell using viral transduction, i.e., by infecting the cell with a virus capable of expressing the nucleic acid such as an adenoviral or lentiviral vector. Moreover, RNA can be introduced into cells using, e.g., lipid nanoparticles or virus-like particles. The choice of the method for delivering the nucleic acid or protein, e.g. the nucleic acid or the fusion protein of the invention, as well as the integration vector into a cell of interest can depend, inter alia, on the type of cell that is to be modified using the method of the invention. If, for instance, the cell is a primary T cell, electroporation may be the preferred method of transfection.

The nucleic acid encoding a protein with serine integrase activity comprising an amino acid sequence having at least 80% sequence identity to SEQ ID NO: 1 or the protein with serine integrase activity comprising an amino acid sequence having at least 80% sequence identity to SEQ ID NO: 1, e.g., the nucleic acid or the fusion protein of the invention, on the one hand and the DNA comprising the cargo sequence, i.e., the integration vector, on the other hand are preferably delivered into the cell simultaneously. They are thus preferably brought into contact with the cell simultaneously or substantially simultaneously, i.e. at an interval of less than 10 minutes, preferably less than 5 minutes or less than 1 minute. Alternatively, there may be a longer time interval between the delivery of the nucleic acid or the protein and the integration vector, e.g. at least 15 minutes, at least 30 minutes, at least 1 hour, at least 2 hours or at least 5 hours, as long as both components are present in the cell at the same time after delivery.

To enable targeted insertion of the cargo sequence into the genomic DNA of the cell, the protein with serine integrase activity comprising an amino acid sequence having at least 80% sequence identity to SEQ ID NO: 1, e.g., the fusion protein of the invention, must not only bind specifically to the attV site in the integration vector but also to a second attachment site within the genomic DNA. In the context of this invention, this attachment site within the cell genome is also referred to as the genomic attachment site or simply attG site (attachment site in Genome). In other words, the term "genomic attachment site" as used herein refers to a nucleic acid sequence that is either present or is being introduced in the genomic DNA of the cell and that can be recognized and bound by a protein with serine integrase activity comprising an amino acid sequence having at least 80% sequence identity to SEQ ID NO: 1 to enable serine integrase-mediated recombination.

The attV site in the integration vector and the attG site in the genome of the cell thus act as pair of recombination sites that allow the introduction of large genomic insertions by the large serine integrase because binding of the enzyme to both the attV and the attG sites results in a strand exchange reaction between the integration vector and the genome that involves double-strand cleavage at the central dinucleotide core of the two attachment sites.

In the context of the present invention, also the attG site is thus derived from either the native Pa01 attP or attB site. However, the attG site in the genome and the attV site in the integration vector must "correspond" to each other to enable serine integrase-mediated recombination via the recombination mechanism described herein. In the context of the invention, the attG site and the attV are considered to "correspond" or to be "corresponding", if i) one of them, e.g., the attV site, is derived from the natural Pa01 attB site as described herein (SEQ ID NO: 26-29), whereas the other, e.g., the attG site, is derived from the natural attP site of Pa01 (SEQ ID NO: 30, 69-71) or vice versa and if ii) the nucleotides forming the dinucleotide core in both attachment sites are identical in the attV and the attG sites when read in 5'-3' direction, so that the serine integrase can mediate the recombination between the attG site and the attV site when these two attachment sites are brought into contact with each other during the recombination process, as described herein.

Thus, in embodiments in which the attV site in the DNA comprising the cargo sequence, i.e., the integration vector, comprises or consists of a nucleic acid sequence having at least 90% sequence identity to any of SEQ ID NO: 26, 27, 28 or 29 (all derived from the Pa01 attB site), the "corresponding" attG site in the genome must comprise or consist of a nucleic acid having at least 90%, at least 95%, at least 99% or 100% sequence identity to any of SEQ ID NO: 30, 69, 70 or 71 (derived from the Pa01 attP site). Likewise, in embodiments in which the attV site in the DNA comprising the cargo sequence, i.e., the integration vector, comprises or consist of a nucleic acid sequence having at least 90% sequence identity to any of SEQ ID NO: 30, 69, 70 or 71, the "corresponding" attG site in the genome must comprise or consist of a nucleic acid having at least 90%, at least 95%, at least 99% or 100% sequence identity to any of SEQ ID NO: 26, 27, 28 or 29.

At least for Bxb1 and other well-established serine integrases, it has been reported that mutations in the central dinucleotide core of the attB and attP sites can be used to produce pairs of orthogonal sites that will selectively recombine only with each other. Using multiple of these orthogonal attachment sites enables a single serine integrase to direct the insertion of multiple cargos, each into a particular landing pad site specified by its cognate core dinucleotide. Use of two orthogonal attachment site pairs in the integration vector and in the genome can also facilitate recombination mediated cassette exchange (RMCE) (Inniss et al., 2017). Multiple orthogonal attachment site pairs can also be used for serine integrase-mediated directional recombination (SIDR) / serine integrase recombinational assembly (SIRA) for larger gene assembly (Colloms et al., 2014). The respective DNA cargo can be delivered as circular DNA or linear DNA (Fauser et al., 2024, https://doi.org/10.1101/2024.05.09.593242).

Accordingly, in preferred embodiments, the native core dinucleotides TC in the naturally occurring (native) attachment sites attB and attP of Pa01 can be mutated (i.e., substituted) to any nucleotides of choice to generate orthogonal attachment sites, e.g., for applications of the present invention combining two or more attachment pairs, such as RMCE or SIDR.

Therefore, the DNA comprising the cargo sequence, i.e., the integration vector used in the method of the present invention, may preferably comprise one or more, e.g., two, three, four, or five attV sites that comprise or consist of a minimal consensus sequence that has been derived from the attB site of Pa01 and has a nucleic acid sequence having at least 90%, e.g., at least 95% or at least 99% sequence identity to CCGTGACCTACATGCX₁X₂GAAGGGCGTATGCGCC (SEQ ID NO: 31) wherein X₁ and X₂ form the central dinucleotide core of this attachment site and can be selected independently of each other from any of A, G, T or C. The one or more attV sites may also comprise or consist of a nucleic acid sequence having 100% sequence identity to SEQ ID NO: 31. The consensus sequence may also be slightly longer, e.g., it may have a nucleic acid sequence with at least 90%, at least 95% or at least 99% sequence identity to GACCGTGACCTACATGCX₁X₂GAAGGGCGTATGCGCCACGAA (SEQ ID NO: 32). The attV site may also comprise or consist of a nucleic acid sequence having 100% sequence identity to SEQ ID NO: 32. Of course, the attV site may also still be longer than any of SEQ ID NO: 31 or 32, e.g., it may comprise or consist of a consensus nucleic acid sequence having at least 90%, at least 95% or at least 95% sequence identity to GCACCGCGGCTTCGAGACCGTGACCTACATGCX₁X₂GAAGGGCGTATGCGCCACGAAGAC CACCTCG (SEQ ID NO: 33), wherein X₁ and X₂ can be selected independently of each other from any of A, G, T or C. It may also comprise or consist of a nucleic acid sequence having 100% sequence identity to SEQ ID NO: 33. The consensus sequence may also have the same length as the naturally occurring attB site of Pa01 and may therefore comprise or consist of a nucleic acid sequence having at least 90%, at least 95% or at least 99% sequence identity to CCGGTTTCCCTTCGCACCCGCACCGCGGCTTCGAGACCGTGACCTACATGCX₁X₂GAAGGG CGTATGCGCCACGAAGACCACCTCGGCAATCGCGGCCTGCTCAAG (SEQ ID NO: 34), wherein X₁ and X₂ can be selected independently of each other from any of A, G, T or C. Again, the attV site may also comprise or consist of a nucleic acid sequence having at least 100% sequence identity to SEQ ID NO: 34.

Thus, the integration vector used in the method of the present invention may preferably comprise one or more, e.g., two, three, four, or five attV sites that comprise or consist of at least a minimal sequence having at least 90% sequence identity to any of CCGTGACCTACATGCAGGAAGGGCGTATGCGCC (SEQ ID NO: 35), CCGTGACCTACATGCACGAAGGGCGTATGCGCC (SEQ ID NO: 36), CCGTGACCTACATGCATGAAGGGCGTATGCGCC (SEQ ID NO: 37), CCGTGACCTACATGCAAGAAGGGCGTATGCGCC (SEQ ID NO: 38), CCGTGACCTACATGCTGGAAGGGCGTATGCGCC (SEQ ID NO: 39), CCGTGACCTACATGCTCGAAGGGCGTATGCGCC (SEQ ID NO: 40), CCGTGACCTACATGCTTGAAGGGCGTATGCGCC (SEQ ID NO: 41), CCGTGACCTACATGCTAGAAGGGCGTATGCGCC (SEQ ID NO: 42), CCGTGACCTACATGCCGGAAGGGCGTATGCGCC (SEQ ID NO: 43), CCGTGACCTACATGCCCGAAGGGCGTATGCGCC (SEQ ID NO: 44), CCGTGACCTACATGCCTGAAGGGCGTATGCGCC (SEQ ID NO: 45), CCGTGACCTACATGCCAGAAGGGCGTATGCGCC (SEQ ID NO: 46), CCGTGACCTACATGCGGGAAGGGCGTATGCGCC (SEQ ID NO: 47), CCGTGACCTACATGCGCGAAGGGCGTATGCGCC (SEQ ID NO: 48), CCGTGACCTACATGCGTGAAGGGCGTATGCGCC (SEQ ID NO: 49), or CCGTGACCTACATGCGAGAAGGGCGTATGCGCC (SEQ ID NO: 50). Optionally, the one or more attV sites comprise or consist of a nucleic acid having at least 95% or at least 99% sequence identity to any of SEQ ID NO: 35-50. In some embodiments, the one or more attV sites comprise a nucleic acid having 100% sequence identity to any of SEQ ID NO: 35-50 or consist thereof.

Alternatively, the DNA comprising the cargo sequence, i.e., the integration vector used in the method of the present invention, may comprise one or more, e.g., two, three, four, or five attV sites that comprises or consist of a minimal consensus sequence that has been derived from the attP site of Pa01 and has a nucleic acid sequence having at least 90% sequence identity to ATCTTGAGTCTTCTTTCX₁X₂GCAAGACAACACGAAAT (SEQ ID NO: 51) wherein X₁ and X₂ form the central dinucleotide core of the attachment site and can be selected independently of each other from any of A, G, T or C. The consensus sequence may also be longer, e.g., it may have a nucleic acid sequence having at least 90% sequence identity to TCATATCCATCTTGAGTCTTCTTTCX₁X₂GCAAGACAACACGAAATAGACACAG (SEQ ID NO: 72) or
CGAGAAAGCAGATTCTCATATCCATCTTGAGTCTTCTTTCX₁X₂GCAAGACAACACGAAATAG ACACAGTCTCTTCCCTAGCTG (SEQ ID NO: 73). It may also have the same length as the naturally occurring attP site of Pa01 and may therefore comprise or consist of a nucleic acid sequence having at least 90% sequence identity to GTAACGCTCTTCGAGAAAGCAGATTCTCATATCCATCTTGAGTCTTCTTTCX₁X₂GCAAGACA ACACGAAATAGACACAGTCTCTTCCCTAGCTGTACACTGAGCC (SEQ ID NO: 52). In all of these consensus sequences 51, 52, 72 and 73, X₁ and X₂ form the dinucleotide core and can be selected independently of each other from any of A, G, T or C. The attV site derived from the attP site of Pa01 may also comprise or consist of a nucleic acid sequence having at least 95% or at least 99% sequence identity to any of SEQ ID NO: 51, 52, 72 or 73. It may also comprise or consist of a nucleic acid having 100% sequence identity to any of SEQ ID NO: 51, 52, 72 or 73.

Thus, the integration vector used in the method of the present invention may also comprise one or more, e.g., two, three, four, or five attV sites that comprise or consist of at least a minimal sequence having at least 90% sequence identity to any of ATCTTGAGTCTTCTTTCAGGCAAGACAACACGAAAT (SEQ ID NO: 53), ATCTTGAGTCTTCTTTCACGCAAGACAACACGAAAT (SEQ ID NO: 54), ATCTTGAGTCTTCTTTCATGCAAGACAACACGAAAT (SEQ ID NO: 55), ATCTTGAGTCTTCTTTCAAGCAAGACAACACGAAAT (SEQ ID NO: 56), ATCTTGAGTCTTCTTTCTGGCAAGACAACACGAAAT (SEQ ID NO: 57), ATCTTGAGTCTTCTTTCTCGCAAGACAACACGAAAT (SEQ ID NO: 58), ATCTTGAGTCTTCTTTCTTGCAAGACAACACGAAAT (SEQ ID NO. 59), ATCTTGAGTCTTCTTTCTAGCAAGACAACACGAAAT (SEQ ID NO: 60), ATCTTGAGTCTTCTTTCCGGCAAGACAACACGAAAT (SEQ ID NO: 61), ATCTTGAGTCTTCTTTCCCGCAAGACAACACGAAAT (SEQ ID NO: 62), ATCTTGAGTCTTCTTTCCTGCAAGACAACACGAAAT (SEQ ID NO: 63), ATCTTGAGTCTTCTTTCCAGCAAGACAACACGAAAT (SEQ ID NO: 64), ATCTTGAGTCTTCTTTCGGGCAAGACAACACGAAAT (SEQ ID NO: 65), ATCTTGAGTCTTCTTTCGCGCAAGACAACACGAAAT (SEQ ID NO: 66), ATCTTGAGTCTTCTTTCGTGCAAGACAACACGAAAT (SEQ ID NO: 67), or ATCTTGAGTCTTCTTTCGAGCAAGACAACACGAAAT (SEQ ID NO: 68). Optionally, the one or more attV sites comprise or consist of a nucleic acid having at least 95% or at least 99% sequence identity to any of SEQ ID NO: 53-68. In some embodiments, the one or more attV sites comprise a nucleic acid having 100% sequence identity to any of SEQ ID NO: 53-68 or consist thereof.

Again, to enable targeted insertion of the cargo sequence into the genomic DNA of the cell, the protein with serine integrase activity comprising an amino acid sequence having at least 80% sequence identity to SEQ ID NO: 1, e.g., the fusion protein of the invention, must bind specifically to one or more genomic attachment sites (attG sites) within the genome of the cell that is to be modified, which are as well derived from the native Pa01 attB site (SEQ ID NO: 31-50) or attP site (SEQ ID NO: 51-68, 72, 73). Thus, the attG site may be derived from the native Pa01 attB site and thus comprise or consist of a nucleic acid sequence having at least 90%, at least 95% or at least 99% sequence identity to any of SEQ ID NO: 31-50. It may also comprise or consist of a nucleic acid sequence having 100% sequence identity to any of SEQ ID NO: 31-50. Alternatively, the attG site may be derived from the native Pa01 attP site and thus comprise or consist of a nucleic acid sequence having at least 90%, at least 95% or at least 99% sequence identity to any of SEQ ID NO: 51-68, 72 or 73. It may also comprise or consist of a nucleic acid sequence having 100% sequence identity to any of SEQ ID NO: 51-68, 72 or 73.

However, if attV sites are used in the integration vector that are derived from the native Pa01 attB or attP sites but in which the dinucleotide core has been mutated to any nucleotides of choice as described herein, the attG sites in the genome of the cell that is to be modified must again "correspond" to the attV sites in the integration vector to enable serine integrase-mediated recombination. Also here, an attV site and an attG site are considered to "correspond" with each other, if i) one of them, e.g., the attV site, is derived from the natural Pa01 attB site as described herein (SEQ ID NO: 31-50), whereas the other, e.g., the attG site, is derived from the natural attP site of Pa01 (SEQ ID NO: 51-68, 72, 73) or vice versa and if ii) X₁ and X₂ forming the dinucleotide core in both attachment sites are identical in the attV and the attG sites when read in 5'-3' direction, so that the serine integrase can mediate the recombination between the attG site and the attV site when these two attachment sites are brought into contact with each other during the recombination process as described herein.

For instance, if an attV site in the integration vector comprises a nucleic acid sequence of SEQ ID NO: 31 derived from the attB site in which, e.g., X₁ is A and X₂ is T (SEQ ID NO: 37), the attG site in the genome of the cell can only be considered to be "corresponding" if it is derived from the attP site, i.e., if it comprises or consist of a nucleic acid sequence of SEQ ID NO: 51 and if X₁ and X₂ of SEQ ID NO: 51 are as well A and T, respectively (SEQ ID NO: 55).

Of course, if the integration vector and the genome of the cell comprise more than one attV and attG site, respectively, it is not necessary that all the attV sites in the integration vector must be, e.g., derived from the natural attB site of Pa01 and all attG sites in the genome must be derived from the natural attP site of Pa01. For instance, the integration vector may as well comprise combinations of different attV sites derived from Pa01's native attB site (e.g., SEQ ID NO: 31-50) and from Pa01's native attP site (SEQ ID NO: 51-68, 72, 73). In such a scenario, the genome of the cell that is to be modified using the method of the invention must comprise corresponding attG sites as defined herein.

In some embodiments of the invention, the genomic DNA of the cell may naturally comprise one or more attG sites as defined herein. In such embodiments, it is thus sufficient to contact the cell with the nucleic acid encoding a protein with serine integrase activity comprising an amino acid sequence having at least 80% sequence identity to SEQ ID NO: 1 (e.g., the nucleic acid of the invention) or the protein with serine integrase activity comprising an amino acid sequence having at least 80% sequence identity to SEQ ID NO: 1 (e.g., the fusion protein of the invention) as well as the integration vector comprising one or more corresponding attV sites as defined herein to effect a targeted insertion of the cargo sequence into the genomic DNA of the cell.

However, typically, the genomic DNA of the cell does not naturally comprise an attG site to which the serine integrase, e.g., the fusion protein of the invention, can bind. Rather, in preferred embodiments of the invention, one or more attG sites comprising or consisting of at least a minimal consensus sequence having at least 90% sequence identity to any of SEQ ID NO: 31 or SEQ ID NO: 51, as disclosed herein, are site-specifically inserted at a specific region of choice within the genomic DNA of the cell. This way, the location of the insertion of the cargo sequence by, e.g., the fusion protein of the invention can be controlled and it can be ensured that cargo integration by the method of the invention does not occur randomly and undesirably within genes or regulatory elements within the genomic DNA of the cell. Accordingly, the risk for, e.g., malignant transformations of cells can be reduced. In some embodiments, it may however also be preferred to site-specifically target a gene of interest within the genomic DNA of the cell using the method of the invention.

The method according to the invention thus optionally comprises a step in which an attG site (herein also referred to as "landing pad") comprising or consisting of at least a minimal consensus sequence having at least 90% sequence identity to SEQ ID NO: 31 or SEQ ID NO: 51 as defined herein is inserted into a desired position within the genomic DNA of the cell. The attG site may of course also comprise or consist of a nucleic acid sequence having at least 90% sequence identity to any of the other herein disclosed attachment sites derived from the native Pa01 attB site (SEQ ID NO: 26-29, 32-50) or attP site (SEQ ID NO: 52-73), as disclosed herein. However, the shorter the attG site, the easier it can be inserted into the genomic DNA, e.g., by homology-directed repair methods. Accordingly, in a preferred embodiment, the attG site that is to be optionally inserted into the genomic DNA of the cell consists of a nucleic acid sequence having at least 90% sequence identity to SEQ ID NO: 31 or SEQ ID NO: 51. The choice of the at least one attG site that is integrated into the genome of the cell depends on the selection of the at least one attV site used in the integration vector and vice versa, because the attG and the attV sites must be selected so that they "correspond" to each other, as defined herein.

Preferably, this optional step of inserting at least one attG site into the genomic DNA of the cell may occur before the step of contacting the cell with the nucleic acid encoding a protein with serine integrase activity comprising an amino acid sequence having at least 80% sequence identity to SEQ ID NO: 1 (e.g., the nucleic acid of the invention) or with the protein with serine integrase activity comprising an amino acid sequence having at least 80% sequence identity to SEQ ID NO: 1 (e.g., the fusion protein of the invention) and the integration vector. However, these steps can also be performed simultaneously or substantially simultaneously, i.e. within less than 10 min, preferably less than 5 min or less than 1 min or at the same time. However, in some embodiments and applications of the invention, it is also possible that much more time passes between step a (integration of the attG site) and step b (recombination with an attV-containing integration vector), for instance during cell line development, in which first a master cell line comprising the inserted attG site is produced, which can then be contacted with, e.g., a fusion protein of the invention and the integration vector at a later time to obtain a new daughter cell line.

The skilled person is well aware of various methods and tools for inserting short foreign nucleic acid sequences such as the herein disclosed attG sequences at a desired position within the genomic DNA of the cell. These methods may involve, e.g., the use of site-specific gene editors, such as site-specific nucleases including, e.g., Zinc finger nucleases (ZFN), Transcription activator-like effector nucleases (TALEN) or CRISPR/Cas for delivering site-specific double-strand breaks (DSBs) into the genome. An exogenous DNA repair template comprising the attG site may then be inserted into the breakage site during homology directed repair (HDR) of the DSB. A more recent and sophisticated method for mediating insertions of short sequences into genomic DNA is known as prime editing. In prime editing, a fusion protein consisting of a catalytically impaired Cas9 endonuclease (H840A nickase) and a moloney murine leukemia virus (M-MLV) reverse transcriptase enzyme is recruited to a target site within the genomic DNA using a prime editing guide RNA (pegRNA). The pegRNA not only serves to detect the target sequence to be edited within the genomic DNA, but also encodes the new genetic information that is to replace the targeted sequence. In the context of the present invention, the pegRNA thus provides the attG sequence comprising or consisting of a nucleic acid having at least 90% sequence identity to e.g. SEQ ID NO: 31 or SEQ ID NO: 51 that is to be specifically integrated into the genomic DNA of the cell. When the fusion protein has been guided by the pegRNA to the target site within the genomic DNA of the cell, it produces a nick in the target DNA sequence, thereby exposing a 3' hydroxyl group that can be used to initiate the reverse transcription of the reverse transcriptase template portion of the pegRNA. This results in a branched intermediate that contains two DNA flaps: a 3' flap that contains the newly synthesized (edited) sequence, and a 5' flap that contains the dispensable, unedited DNA sequence. The 5' flap is then cleaved by structure-specific endonucleases or 5' exonucleases. This process allows for 3' flap ligation and creates a heteroduplex DNA composed of one edited strand and one unedited strand. The reannealed double stranded DNA contains nucleotide mismatches at the location where editing took place. In order to correct the mismatches, the cells exploit the intrinsic mismatch repair (MMR) mechanism, with two possible outcomes: (i) the information in the edited strand is copied into the complementary strand, permanently installing the edit; (ii) the original nucleotides are re-incorporated into the edited strand, excluding the edit (https://en.wikipedia.org/wiki/Prime_editing). For the sake of simplicity, the above-described gene editing system comprising the pegRNA and the H840A nickase fused to the reverse transcriptase enzyme suitable for performing prime editing is herein referred to as "prime editor".

Of course, there are various other gene editing methods and tools known to the skilled person that can be used for target-specifically integrating one or more attG sites into the genome of a cell. These methods include click editing or similar templated gene editing methods that use DNA-polymerases, bridge editing or the use of CRISPR-associated transposons (CAST).

Click editing is a method that resembles prime editing but relies on the enzymatic activity of DNA-dependent polymerases rather than reverse transcriptase. Click editing is described, e.g., in Ferreira da Silva et al., 2024 or Liu et al., 2024. In brief, click editing typically involves the use of a fusion protein consisting of an RNA-programmed DNA nickase, a DNA-dependent DNA polymerase, and a ssDNA tethering domain (e.g. an HUH endonuclease; HUHe) paired with a guide RNA (gRNA). The system leverages the "click-like" protein-substrate attachment biochemistry of HUHes to localize ssDNA oligo template "click DNAs" (clkDNAs) for precision genome editing (Ferreira da Silva et al., 2024). Systems suitable for performing site-specific "click-editing" of a target DNA as disclosed in, e.g., Ferreira da Silva et al., 2024 or Liu et al., 2024 are herein collectively summarized by the term "click editor".

Bridge editing relies on the use of autonomous genetic elements known as IS110 insertion sequences that encode a specific recombinase as well as express a structured non-coding RNA (known as bridge RNA). The bridge RNA can bind specifically to the recombinase encoded by the IS110 element and further comprises two internal loops encoding nucleotide stretches that base-pair with a target DNA and a donor DNA, the latter being the IS110 element itself. Both the target- and the donor-binding loops of the bridge RNA can be independently reprogrammed to direct sequence-specific recombination between two DNA molecules of choice and thus to enable the insertion of DNA into genomic target sites (Durrant et al., 2024). For the sake of simplicity, the above-described gene editing system comprising the bridge RNA and the IS110 recombinase suitable for performing bridge editing is herein referred to as "bridge editor".

CRISPR-associated transposons (CASTs) are mobile genetic elements (MGEs) that have evolved to make use of minimal CRISPR systems for RNA-guided transposition of their DNA. Unlike traditional CRISPR systems that contain interference mechanisms to degrade targeted DNA, CASTs lack proteins and/or protein domains responsible for DNA cleavage. Instead, a specialized transposon machinery, similar to that of the well characterized Tn7 transposon which functions with a cut and paste mechanism, complexes with the CRISPR RNA (crRNA) and associated Cas proteins for transposition (https://en.wikipedia.org/wiki/CRISPR-associated_transposons).

In some embodiments of the method of the invention, the site-specific insertion of the attG site into the genomic DNA and the subsequent insertion of the cargo sequence can be both achieved using the fusion protein of the invention. This requires the use of a fusion protein as described herein, which comprises a large serine integrase consisting of a protein having at least 80% sequence identity to SEQ ID NO: 1 and, all fused to the C-terminus of said serine integrase, a gene editor comprising a CRISPR-Cas9 nickase, a reverse transcriptase (RT) and the NLS. Such a fusion protein may thus resemble a genome editing tool called PASTE ("programmable addition via site-specific targeting elements") as disclosed in WO 2022087235 A1 and Yarnall et al., 2023.

Site-specific insertion of the one or more attG sites as defined herein makes it possible to later-on insert the cargo sequence at any target locus of interest within the genomic DNA of the cell using the herein disclosed method of the invention. The choice of the specific locus that is to be modified with the inventive method largely depends on the type of modification that is to be introduced into the cell, the cell type used or the desired application of the engineered cell. For instance, in one embodiment, if the cell that is to be modified using the method of the present invention is a T cell, the locus that may be targeted can be the gene encoding the T-cell surface glycoprotein CD3 zeta chain. It may also be, e.g., the locus that comprises the GAPDH gene.

Also disclosed herein is an engineered cell obtainable by the method of the invention. Such a cell is characterized by the presence of the cargo sequence as well as specific attL and attR sequences flanking said cargo sequence. The attL and attR sequences are created by the genetic rearrangements resulting from the specific interaction of the protein with serine integrase activity comprising an amino acid sequence having at least 80% sequence identity to SEQ ID NO: 1 (e.g., the fusion protein according to the invention) with the attG and attV sites within the genomic DNA of the cell and the integration vector, respectively. The specific attL and the attR sequences in the genome of the cell are thus uniquely determined by the choice of the precise attV and corresponding attG sites during the method of the invention. The presence of the attL and attR sequences in the engineered cell can be determined by the skilled person using standard laboratory methods such as sequencing or polymerase chain reaction (PCR) using suitable primers.

The present invention also provides a cell comprising the nucleic acid and/or fusion protein of the invention, wherein the cell preferably also expresses the fusion protein of the invention. The cell can be a bacterial cell, e.g., *E. coli*, but it is preferably a eukaryotic cell, e.g., a mammalian cell. Typically, for research purposes, the cell is a mouse or human cell. Therapeutic cells are usually human cells, and a human subject is to be treated. The cell may, e.g., be a T cell, NK cell or NKT cell, most preferably, a T cell.

In another aspect, the present invention also provides a first composition comprising:
a) a nucleic acid encoding a protein with serine integrase activity comprising an amino acid sequence having at least 80% sequence identity to SEQ ID NO: 1 or a protein with serine integrase activity comprising an amino acid sequence having at least 80% sequence identity to SEQ ID NO: 1, or a cell comprising said nucleic acid and/or said protein,
b) a DNA comprising at least one vector attachment site comprising or consisting of a nucleic acid sequence having at least 90% sequence identity to any of SEQ ID NO: 31 or 51 as defined herein and a cargo sequence,
c) a source for a gene editor capable of site-specifically introducing at least one genomic attachment site into the genomic DNA of a cell,
d) a nucleic acid providing at least one genomic attachment site comprising or consisting of a nucleic acid sequence having at least 90% sequence identity to any of SEQ ID NO: 31 or 51 as defined herein that corresponds to the at least one vector attachment site in the DNA according to b) to enable serine integrase-mediated recombination, and
e) optionally, at least one carrier and/or excipient.

Preferably, the first composition comprises
a) the nucleic acid of the invention, the fusion protein of the invention, or a cell comprising the nucleic acid and/or fusion protein of the invention, as disclosed above,
b) a DNA comprising at least one vector attachment site comprising or consisting of a nucleic acid sequence having at least 90% sequence identity to any of SEQ ID NO: 31 or 51 as defined herein and a cargo sequence,
c) a source for a gene editor capable of site-specifically introducing at least one genomic attachment site into the genomic DNA of a cell,
d) a nucleic acid providing at least one genomic attachment site comprising or consisting of a nucleic acid sequence having at least 90% sequence identity to any of SEQ ID NO: 31 or 51 as defined herein that corresponds to the at least one attV site in the DNA according to b) to enable serine integrase-mediated recombination, and
e) optionally, at least one carrier and/or excipient.

Preferably, the first composition is a first pharmaceutical composition, i.e., the cargo sequence encodes a gene that, when expressed by a cell, can exert a therapeutic effect. The cargo sequence may, e.g., encode a T cell receptor or a CAR with specificity for, e.g., a cancer antigen or an intact copy of a gene, e.g., a proto-oncogene or a tumor suppressor gene that is to replace a defective copy of the same gene in a target cell, or an intact copy of a gene encoding an enzyme defective in a hereditary disease such as cystic fibrosis or a muscular dystrophy. The cargo sequence may also encode a non-coding RNA, such as a microRNA or a long non-coding RNA.

In a preferred embodiment, the composition comprises a nucleic acid encoding a protein with serine integrase activity comprising an amino acid sequence having at least 80% sequence identity to SEQ ID NO: 1, e.g., a nucleic acid according to the invention encoding the fusion protein. Preferably the nucleic acid is an in vitro-transcribed mRNA or an expression vector, e.g., a viral vector as described herein. If the nucleic acid is an mRNA, it is preferably present in the composition within a suitable delivery vehicle. The delivery vehicle may e.g., comprise a lipid membrane that encapsulates the inhibitory oligonucleotide. For example, the vehicle may be a liposomal or an exosomal carrier comprising the mRNA as a cargo and, optionally, expressing a targeting moiety on its surface. The delivery vehicle may also be a lipid nanoparticle, a bacterial minicell, an extracellular vesicle, or a micro- or nanoparticle formed by a cationic polymer such as e.g., polyethylenimine (PEI) or DEAE-dextran.

In the context of the present invention, the term "gene editor" refers to any protein, fusion protein or system that is capable of inserting an attG site as described herein, i.e., a sequence comprising or consisting of a nucleic acid sequence having at least 90% sequence identity to any of SEQ ID NO: 31 or SEQ ID NO: 51 into the genome of a target cell. The gene editor may thus be, e.g., a site-specific nuclease or nickase, a CRISPR-associated transposon, a prime editor, a click editor or a bridge editor, as described herein. The source for the gene editor, such as a site-specific nuclease or nickase capable of site-specifically introducing at least one attG site into the genomic DNA of a cell can be a protein. Preferably, it is a Cas nuclease combined with a suitable guide RNA (e.g., a sgRNA) that targets the Cas nuclease to a site of interest within the genome of a cell. It may also be a prime editor, a click editor, a bridge editor or a CRISPR-associated transposon (CAST) as described herein.

For instance, if the gene editor is a site-specific nuclease such as a Cas nuclease combined with a suitable gRNA, the nucleic acid providing the at least one attG site that corresponds to the at least one attV site in the DNA according to b) serves as a DNA repair template that can be inserted at a cleavage site induced by the site-specific nuclease via homology-directed repair. In such an embodiment, said nucleic acid of d) thus is a DNA molecule comprising at least one attG site as disclosed herein that corresponds to the at least one attV site in the DNA according to b) and which preferably further comprises so-called homology arms, i.e., nucleic acid regions flanking the at least one attG site that share homology with the genomic locus into which the at least one attG is to be inserted via homology-directed repair. In embodiments where a gene editor is used that does not rely on homology-directed repair for introducing the at least one attG site as described herein, e.g., a prime editor, a click editor, a bridge editor or a CAST, the skilled person will have no problem of designing the nucleic acid of d) so that it can be utilized by the respective gene editor. Thus, depending on the gene editor that is used in the pharmaceutical composition, the nucleic acid providing the at least one attG site as disclosed herein that corresponds to the at least one attV site in the DNA according to b) can be single-stranded or double-stranded DNA or RNA. Moreover, in the context of the present invention, the term "a nucleic acid providing at least one genomic attachment site..." is intended to refer to either a nucleic acid that comprises a nucleic acid sequence having at least 90% sequence identity to any of SEQ ID NO: 31 or 51 or a nucleic acid that comprises a sequence which is complementary to a nucleic acid sequence having at least 90% sequence identity to any of SEQ ID NO: 31 or 51. The latter may, e.g., be a an RNA template for reverse transcriptase, e.g., a pegRNA of a prime editor.

Thus, in a particular preferred embodiment, the gene editor can be a Cas9 nickase fused to a moloney murine leukemia virus (M-MLV) reverse transcriptase enzyme suitable for performing prime editing as described herein. In this case, the nucleic acid providing an attG site comprising or consisting of a nucleic acid sequence having at least 90% sequence identity to any of SEQ ID NO: 31 or 51, as defined herein, is a prime editing guide RNA (pegRNA) which not only provides the repair template, but also serves to recruit the nuclease to a target sequence with a host cell genome.

In some embodiments, the source of the gene editor provided by the composition may also be a nucleic acid encoding said gene editor, e.g., an mRNA or an expression vector. Said nucleic acid can be a separate nucleic acid, i.e., it is a different nucleic acid than the nucleic acid encoding a protein with serine integrase activity, e.g., the fusion protein of the invention. In alternative embodiments, the same nucleic acid which encodes, e.g., the fusion protein of the invention may also encode the gene editor.

In a particular preferred embodiment, the composition comprises a fusion protein as disclosed herein, which comprises a large serine integrase consisting of a protein having at least 80% sequence identity to SEQ ID NO: 1 and, fused to the C-terminus of said serine integrase, a CRISPR-Cas9 nickase, a reverse transcriptase (RT) and the NLS, as described herein. The composition may alternatively comprise a nucleic acid encoding such a fusion protein. In such an embodiment, the composition does not comprise a separate source for a gene editor, because said site-specific gene editor is already part of the fusion protein.

In a further embodiment, the present invention also provides a second pharmaceutical composition comprising an engineered cell obtainable by the above-described method of the invention, e.g., an engineered T cell or stem cell.

Any of the herein disclosed compositions may further comprise one or more carriers such as buffers, e.g., physiological saline or PBS. They may also comprise one or more delivery vehicles for delivering the different components of the compositions, e.g., lipid nanoparticles, virus-like particles, nano polymers, lipids, viruses or vectors, as already described elsewhere herein. The compositions may also comprise one or more excipients, e.g., stabilizers such as SPGA, carbohydrates (e.g. sorbitol, mannitol, starch, sucrose, glucose, dextran), proteins such as albumin or casein, or protein containing agents such as bovine serum or skimmed milk. If the composition does not comprise cells but only nucleic acids and/or proteins, it can be a dry composition, e.g., a lyophilized composition. Such compositions typically comprise a bulking agent, e.g., a nonreducing sugar such as trehalose. They may also comprise a buffering agent.

Further, the invention provides a kit comprising
a) the nucleic acid of the invention, the fusion protein of the invention, and/or the cell of the invention;
b) a DNA integration vector comprising at least one vector attachment site (attV site) comprising or consisting of a nucleic acid sequence having at least 90% sequence identity to any of SEQ ID NO: 31 or SEQ ID NO: 51, one or more restrictions sites for introducing a cargo nucleic acid sequence of interest and, optionally, a selective marker.

Such a kit may be used for delivering a cargo nucleic acid of interest into a genome of a cell, e.g., a human cell. The kit may optionally comprise further components such as gene editors and/or nucleic acids encoding a gene editor as disclosed herein. The kit may also comprise a template nucleic acid providing at least one attG site as defined herein that corresponds to the at least one attV site in the integration vector and that is to be inserted into the genome of a target cell using the gene editor. The kit may be used for *in vivo*, *ex vivo* or *in vitro* applications.

Dependent on the cargo sequence that is inserted into the DNA integration vector, the kit may also be a pharmaceutical kit.

Thus, in another aspect, the present invention also provides a pharmaceutical kit comprising
a) a nucleic acid encoding a protein with serine integrase activity comprising an amino acid sequence having at least 80% sequence identity to SEQ ID NO: 1 (e.g., a nucleic acid of the present invention) or a protein with serine integrase activity comprising an amino acid sequence having at least 80% sequence identity to SEQ ID NO: 1 (e.g., a fusion protein of the present invention), or a cell comprising said nucleic acid and/or said protein,
b) a DNA comprising at least one vector attachment site comprising or consisting of a nucleic acid sequence having at least 90% sequence identity to any of SEQ ID NO: 31 or 51 as defined herein and a cargo sequence,
c) a source for a gene editor capable of site-specifically introducing at least one genomic attachment site into the genomic DNA of a cell,
d) a nucleic acid providing at least one genomic attachment site comprising or consisting of a nucleic acid sequence having at least 90% sequence identity to any of SEQ ID NO: 31 or 51 as defined herein that corresponds to the at least one vector attachment site in the DNA according to b) to enable serine integrase-mediated recombination, and
e) optionally, at least one carrier and/or excipient.

The cargo sequence of the DNA of b) is thus a therapeutic cargo sequence, i.e., it encodes a gene that, when expressed by a cell, can exert a therapeutic effect. The cargo sequence may, e.g., encode a T cell receptor or a CAR with specificity for, e.g., a cancer antigen or an intact copy of a gene, e.g., a proto-oncogene or a tumor suppressor gene that is to replace a defective copy of the same gene in a target cell, a non-coding regulatory RNA, or an intact copy of a gene encoding an enzyme defective in a hereditary disease such as cystic fibrosis or a muscular dystrophy.

The different components of the pharmaceutical kit are the same components described above for the first composition. However, the different components are preferably present in the pharmaceutical kit separately from one another, which means they can be administered to a patient separately. Accordingly, each component of the pharmaceutical kit can be provided in a separate delivery vehicle as disclosed herein, which can be specifically chosen, dependent on whether the respective components are nucleic acids or proteins. It may also be possible that the pharmaceutical kit provides several of its components in the same delivery vehicle. For instance, in some embodiments, it may be advantageous if, e.g., the pharmaceutical kit provides a first delivery vehicle which comprises components a) and b) and a second delivery vehicle that comprises components c) and d).

The herein disclosed first composition and kits can be used for various biotechnological applications, e.g. in the production of therapeutically effective cells, in cell line engineering, or the production of protein expression cell lines, e.g., as already described herein.

Likewise, the herein disclosed first or second pharmaceutical composition or the herein disclosed pharmaceutical kit can be for use in treating a disease or disorder in a subject.

Accordingly, the present invention also provides a method for treating a disease or disorder in a subject, comprising a step of administering to the subject a pharmaceutically effective amount of a pharmaceutical composition disclosed herein or a pharmaceutically acceptable amount of the components of the pharmaceutical kit as disclosed herein.

In the context of the invention, "treating a disease or disorder" is to be understood to comprise a curative medical therapy of a subject with the intent to cure, ameliorate or stabilize a condition. In some embodiments, treating a disease or disorder is intended to mean that the progression of the disease or disorder is to be slowed, stopped or, preferably, reversed to allow for a perceivable improvement of the subject's well-being and health. The term "treating a disease or disorder" as used herein also encompasses preventing a disease or disorder, i.e., to precluding, averting, obviating, forestalling, stopping, reducing incidence or severity or hindering said disease or disorder from happening in the first place. The term "treating a disease or disorder" thus also explicitly includes the prophylactic treatment of a subject.

The subject of the herein disclosed invention can be a vertebrate, such as a mammal, a fish, a bird, a reptile, or an amphibian. Preferably, the subject is a mammal, e.g., a human, non-human primate, horse, pig, rabbit, dog, sheep, goat, cow, camel, cat, guinea pig, rat or mouse. In a particularly preferred embodiment, the subject is a human, i.e., a patient.

Because the fusion protein of the invention is capable of site-specifically inserting large cargo sequences of at least up to 1000 bp, at least up to 2000 bp, at least up to 5000 bp, at least up to 8000 bp, at least up to 10000 bp, at least up to 20000 bp, at least up to 30000 bp, of at least up to 40000 bp, at least up to 50000 bp, at least up to 75000 bp and possibly at least up to 100000 bp into the genome of a cell of interest, it is particular suitable for treating a disease that is based on large deletions or many mutations in one or more genes.

The disease can be a cancer such as head and neck cancer, laryngeal and hypopharyngeal cancer, nasal cavity and paranasal sinuses cancer, nasopharyngeal cancer, oral cavity and oropharyngeal cancer, salivary gland cancer, anal cancer, bile duct cancer, colorectal cancer, esophagus cancer, gallbladder cancer, gastrointestinal neuroendocrine (carcinoid) tumors, gastrointestinal stromal tumor (GIST), liver cancer, pancreatic cancer, pancreatic neuroendocrine tumor (NET), small Intestine cancer, stomach cancer, bladder cancer, kidney cancer, Wilms tumor, lung cancer, lung carcinoid tumor, malignant mesothelioma, breast cancer, cervical cancer, endometrial cancer, ovarian cancer, penile cancer, prostate cancer, testicular cancer, uterine sarcoma, vaginal cancer, vulvar cancer, adrenal cancer, gastrointestinal neuroendocrine (carcinoid) tumors, lung carcinoid tumor, pancreatic neuroendocrine tumor (NET), pituitary tumors, thyroid cancer, skin cancer, basal and squamous cell skin cancer, Kaposi sarcoma, lymphoma of the skin, melanoma skin cancer, merkel cell skin cancer, bone cancer, Ewing Family of tumors, osteosarcoma, rhabdomyosarcoma, soft tissue sarcoma, eye cancer, retinoblastoma, brain and spinal cord tumors, neuroblastoma, leukemia, acute lymphocytic leukemia (ALL) in adults, acute myeloid leukemia (AML) in adults, chronic lymphocytic leukemia (CLL), chronic myeloid leukemia (CML), chronic myelomonocytic leukemia (CMML), leukemia in children, lymphoma, non-Hodgkin lymphoma, Hodgkin lymphoma, multiple myeloma, myelodysplastic syndromes, thymus cancer and Waldenstrom macroglobulinemia.

The disease may alternatively also be a chronic inflammatory disorder.

It may, e.g., be an autoimmune disorder such as inflammatory bowel disease, multiple sclerosis, lupus, psoriatic arthritis, rheumatoid arthritis, Sjögren's syndrome, Crohn's disease, celiac disease ulcerative colitis, Grave's disease, Hashimoto's thyroiditis, Addison's disease, dermatomyositis, psoriasis, chronic inflammatory demyelinating polyneuropathy, Guillain-Barre syndrome, myasthenia gravis, autoimmune vasculitis, type 1 diabetes, pernicious anemia or vasculitis , transplant rejection and graft acceptance, graft versus host disease, or host versus graft disease.

In some embodiments, the disease may also be a musculoskeletal disorder (MSD) such as, e.g., carpal tunnel syndrome, epicondylitis, tendinitis, back pain, tension neck syndrome, and hand-arm vibration syndrome.

The disease may also be a cardiovascular disorder, e.g., a heart attack, a cardiomyopathy, an aorta disease, Marfan syndrome, a coronary artery disease, a deep vein thrombosis, a pulmonary embolism, a pericardial disease, a cerebrovascular disease and the like.

The disease may also be an infection or a wound due to, e.g. burns that may require regenerative medicine. For many of the diseases disclosed herein, T cells could be specifically modified for treatment using the herein disclosed compositions and methods.

The disease may also be a hereditary disease caused by one or more mutations in a single gene (monogenic) or multiple genes (polygenic) or by a chromosome abnormality (e.g. a large deletion, inversion or duplication). The mutations may also affect non-coding areas of the genome, e.g., gene-regulatory elements such as gene promoters, enhancers, or silencers or genes encoding regulatory non-coding RNAs such as microRNAs, small interfering RNAs, long non-coding RNAs or circular RNAs. Hereditary disease may be treated using the pharmaceutical compositions of the present invention, e.g., by introducing an intact copy of a deficient gene or genetic locus into the patient's cells.

The hereditary disease may e,g., be a metabolic disease, e.g., a congenital metabolic disease (CMD). The hereditary disease may also e.g., be a congenital heart disease, cystic fibrosis, a muscular dystrophy, a hereditary neurological disorder such as, e.g., Rett-syndrome, a hereditary degenerative disorder, e.g., a neurodegenerative disorder such as dementia, Alzheimer's disease or Parkinson's disease, hemophilia, such as e.g., factor VIII deficiency, factor X deficiency or the like. It may also be a sensory disorder, such a blindness, deafness or dumbness due to genetic causes.

In one embodiment, the herein disclosed first pharmaceutical composition or the herein disclosed pharmaceutical kit can be for use in *in vivo* gene therapy, i.e., the composition or the different components of the kit may be administered in vivo to a cell of interest to modify the genome of said cell and thus to treat a disease or disorder in a subject as defined herein. To do so, the pharmaceutical composition or the different components of the pharmaceutical kit may be administered to a subject either systemically or locally. The exact route of administration depends largely on the specific disease or disorder that is to be treated. For instance, during *in vivo* gene therapy, the pharmaceutical composition or the components of the pharmaceutical kit may be administered parenterally, i.e., via intravenous, intramuscular, subcutaneous or intradermal injection. If the disease that is to be treated affects, e.g., the respiratory tract of the subject to be treated, the pharmaceutical composition or the components of the pharmaceutical kit can be provided in a form so that it can be administered to a subject in need thereof via inhalation, e.g., as an aerosol.

In another embodiment, the above-described first pharmaceutical composition of the invention or the pharmaceutical kit may also be for use in *ex vivo* gene therapy, i.e., they can be used to *ex vivo* modify a cell that has been isolated from a subject to express a gene that has a therapeutic effect, e.g., by using the herein disclosed method of the invention. The thus genetically modified cell is subsequently re-introduced into the subject to exert a therapeutic effect, preferably as part of the second pharmaceutical composition as disclosed herein. Thus, the second pharmaceutical composition can be for use in *ex vivo* gene therapy as well.

If, e.g., in a preferred embodiment, the cell that is to be treated *ex vivo* using the first pharmaceutical composition or the pharmaceutical kit of the invention or the engineered cell present in the second pharmaceutical composition of the invention is a T cell, the *ex-vivo* gene therapy is an adoptive T cell therapy. E.g., engineered CD4+ T cells or CD8 + T cells or gamma delta T cells targeting a tumor antigen can be used. Adoptive T cell therapy may be a CAR-T cell therapy, a transgenic TCR T cell therapy, or a regulatory T cell therapy.

If, in another embodiment, the cell that is to be treated *ex vivo* using the first pharmaceutical composition or the pharmaceutical kit of the invention or the engineered cell present in the second pharmaceutical composition of the invention is a stem cell, the *ex vivo* gene therapy is stem cell therapy.

A stem cell therapy can also be performed as an *in vivo* gene therapy.

The cell that is to be treated *ex vivo* using the first pharmaceutical composition or the pharmaceutical kit of the invention or the engineered cell present in the second pharmaceutical composition of the invention may also be, e.g., an NK cell, an NKT cell, a macrophage, a monocyte, a dendritic cell, a hematopoietic stem cell or progenitor cell, an induced pluripotent stem cell or any type of differentiated somatic cell that has been derived from an induced pluripotent stem cell such as a muscle cell, a nerve cell, a liver cell, a kidney cell, a heart cell or a thymus cell.

There are also non-pharmaceutical applications, e.g., the fusion proteins, methods, compositions and kits of the invention can be used to generate cells, e.g., CHO cells expressing antibody libraries, cells expressing proteins, e.g., with optimal expression, cells expressing complete enzyme systems, e.g., with enzymes of a metabolic pathway, such that the cells can then produce a desired end product, e.g., a small molecule that can be therapeutically used.

Taken together, the present invention provides an improved serine integrase based on Pa01 that is characterized by surprisingly enhanced nuclear delivery and efficient integration of the cargo sequence. This was achieved by fusing a nucleus localization signal to the c-terminal end of the serine integrase. The c-NLS-Pa01 variant can serve as a powerful tool to develop cell and gene therapies that require large cargo delivery, but also other biotechnology solutions such as cell line engineering. The present invention defined the C-terminal end as the position at which a serine integrase having at least 80% sequence identity to SEQ ID NO. 1 can be modified without disrupting its enzymatic function, opening avenues for the generation of fusion proteins for targeted gene delivery.

In particular, the invention provides the following embodiments:
1. A nucleic acid encoding a fusion protein comprising
   a) a serine integrase consisting of a protein having at least 80% sequence identity to SEQ ID NO: 1 and
   b) a nuclear localization signal (NLS) at the C-terminus of the serine integrase.
2. The nucleic acid of embodiment 1, wherein the NLS comprises
   a) at least one NLS comprising a consensus sequence of KX₁X₂X₃, wherein X₁ and X₃ can be either K or R and X₂ can be any amino acid, and/or
   b) at least one NLS comprising a consensus sequence of X₁(X₂)₁₀₋₁₂KRX₃K, where X₁ can be either K or R and X₂ and X₃ can be any amino acid, and/or
   c) at least one NLS selected from the group comprising SEQ ID NO: 10-14.
3. The nucleic acid of embodiment 1 or 2, wherein the NLS comprises at least one NLS comprising a consensus sequence of KX₁X₂X₃, wherein X₁ and X₃ can be either K or R and X₂ can be any amino acid.
4. The nucleic acid of embodiment 1 or 2, wherein the NLS comprises a consensus sequence of X₁(X₂)₁₀₋₁₂KRX₃K, where X₁ can be either K or R and X₂ and X₃ can be any amino acid.
5. The nucleic acid of embodiment 1 or 2, wherein the NLS comprises at least one NLS comprising a nucleic acid sequence of SEQ ID NO: 10.
6. The nucleic acid of embodiment 1 or 2, wherein the NLS comprises at least one NLS of SEQ ID NO: 11.
7. The nucleic acid of embodiment 1 or 2, wherein the NLS comprises at least one NLS of SEQ ID NO: 12.
8. The nucleic acid of embodiment 1 or 2, wherein the NLS comprises at least one NLS of SEQ ID NO: 13.
9. The nucleic acid of embodiment 1 or 2, wherein the NLS comprises at least one NLS of SEQ ID NO: 14.
10. The nucleic acid of any of embodiments 3-9, further comprising at least one additional NLS comprising:
   a) a consensus sequence of KX₁X₂X₃, wherein X₁ and X₃ can be either K or R and X₂ can be any amino acid, and/or
   b) a consensus sequence of X₁(X₂)₁₀₋₁₂KRX₃K, where X₁ can be either K or R and X₂ and X₃ can be any amino acid, and/or
   c) SEQ ID NO: 10-14.
11. The nucleic acid of any of embodiments 1 or 2, wherein the NLS comprises any of SEQ ID NO: 2; SEQ ID NO: 3, SEQ ID NO: 4; SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, or any combination thereof.
12. The nucleic acid of any of embodiments 1 or 2, wherein the NLS comprises SEQ ID NO: 2.
13. The nucleic acid of any of embodiments 1 or 2, wherein the NLS comprises SEQ ID NO: 3.
14. The nucleic acid of any of embodiments 1 or 2, wherein the NLS comprises SEQ ID NO: 4.
15. The nucleic acid of any of embodiments 1 or 2, wherein the NLS comprises SEQ ID NO: 5.
16. The nucleic acid of any of embodiments 1 or 2, wherein the NLS comprises SEQ ID NO: 6.
17. The nucleic acid of any of embodiments 1 or 2, wherein the NLS comprises SEQ ID NO: 7.
18. The nucleic acid of any of embodiments 1 or 2, wherein the NLS comprises SEQ ID NO: 8.
19. The nucleic acid of any of embodiments 1 or 2, wherein the NLS comprises SEQ ID NO: 9.
20. The nucleic acid of any of embodiments 1 or 2, wherein the NLS comprises SEQ ID NO: 10.
21. The nucleic acid of any of embodiments 1 or 2, wherein the NLS comprises SEQ ID NO: 11.
22. The nucleic acid of any of embodiments 1 or 2, wherein the NLS comprises SEQ ID NO: 12.
23. The nucleic acid of any of embodiments 1 or 2, wherein the NLS comprises SEQ ID NO: 13.
24. The nucleic acid of any of embodiments 1 or 2, wherein the NLS comprises SEQ ID NO: 14.
25. The nucleic acid of any of embodiments 1 or 2, wherein the NLS comprises SEQ ID NO: 15.
26. The nucleic acid of any of embodiments 12-25, wherein the NLS further comprises any of SEQ ID NO: 2; SEQ ID NO: 3, SEQ ID NO: 4; SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, or SEQ ID NO: 15.
27. A fusion protein comprising a serine integrase encoded by the nucleic acid of any of embodiments 1-26.
28. A method of preparing an engineered cell comprising a cargo sequence in its genomic DNA, comprising steps of:
   a. optionally, site-specifically inserting one or more genomic attachment sites (attG sites) comprising a nucleic acid sequence having at least 90% sequence identity to any of SEQ ID NO: 31 or SEQ ID NO: 51 into the genomic DNA of a cell, and
   b. contacting the cell with a nucleic acid encoding a protein with serine integrase activity comprising an amino acid sequence having at least 80% sequence identity to SEQ ID NO: 1 or with a protein with serine integrase activity comprising an amino acid sequence having at least 80% sequence identity to SEQ ID NO: 1 and with a DNA comprising i) one or more vector attachment sites (attV sites) comprising a nucleic acid sequence having at least 90% sequence identity to any of SEQ ID NO: 31 or SEQ ID NO: 51 and ii) the cargo sequence,
   wherein the one or more genomic attachment sites and the one or more vector attachment sites correspond to each other to enable serine integrase-mediated recombination and wherein the method can be an *in vivo*, *ex vivo* or *in vitro* method.
29. A pharmaceutical composition comprising
   a) a nucleic acid encoding a protein with serine integrase activity comprising an amino acid sequence having at least 80% sequence identity to SEQ ID NO: 1 or a protein with serine integrase activity comprising an amino acid sequence having at least 80% sequence identity to SEQ ID NO: 1, or a cell comprising said nucleic acid and/or said protein,
   b) a DNA comprising at least one vector attachment site comprising or consisting of a nucleic acid sequence having at least 90% sequence identity to any of SEQ ID NO: 31 or 51 and a cargo sequence,
   c) a source for a gene editor capable of site-specifically introducing at least one genomic attachment site into the genomic DNA of a cell,
   d) a nucleic acid providing at least one genomic attachment site comprising or consisting of a nucleic acid sequence having at least 90% sequence identity to any of SEQ ID NO: 31 or 51 that corresponds to the at least one vector attachment site in the DNA according to b) to enable serine integrase-mediated recombination,
   e) optionally, at least one carrier and/or excipient.
30. The method of embodiment 28 or the pharmaceutical composition of embodiment 29, wherein the genomic attachment site comprises a nucleic acid sequence having at least 90% sequence identity to SEQ ID NO: 51 and the vector attachment site comprises a nucleic acid sequence having at least 90% sequence identity to SEQ ID NO: 31.
31. The method of embodiment 30 or the pharmaceutical composition of embodiment 30, wherein the genomic attachment site comprises a nucleic acid sequence having at least 90% sequence identity to SEQ ID NO: 53 and the vector attachment site comprises a nucleic acid sequence having at least 90% sequence identity to SEQ ID NO: 35.
32. The method of embodiment 30 or the pharmaceutical composition of embodiment 30, wherein the genomic attachment site comprises a nucleic acid sequence having at least 90% sequence identity to SEQ ID NO: 54 and the vector attachment site comprises a nucleic acid sequence having at least 90% sequence identity to SEQ ID NO: 36.
33. The method of embodiment 30 or the pharmaceutical composition of embodiment 30, wherein the genomic attachment site comprises a nucleic acid sequence having at least 90% sequence identity to SEQ ID NO: 55 and the vector attachment site comprises a nucleic acid sequence having at least 90% sequence identity to SEQ ID NO: 37.
34. The method of embodiment 30 or the pharmaceutical composition of embodiment 30, wherein the genomic attachment site comprises a nucleic acid sequence having at least 90% sequence identity to SEQ ID NO: 56 and the vector attachment site comprises a nucleic acid sequence having at least 90% sequence identity to SEQ ID NO: 38.
35. The method of embodiment 30 or the pharmaceutical composition of embodiment 30, wherein the genomic attachment site comprises a nucleic acid sequence having at least 90% sequence identity to SEQ ID NO: 57 and the vector attachment site comprises a nucleic acid sequence having at least 90% sequence identity to SEQ ID NO: 39.
36. The method of embodiment 30 or the pharmaceutical composition of embodiment 30, wherein the genomic attachment site comprises a nucleic acid sequence having at least 90% sequence identity to SEQ ID NO: 58 and the vector attachment site comprises a nucleic acid sequence having at least 90% sequence identity to SEQ ID NO: 40.
37. The method of embodiment 30 or the pharmaceutical composition of embodiment 30, wherein the genomic attachment site comprises a nucleic acid sequence having at least 90% sequence identity to SEQ ID NO: 59 and the vector attachment site comprises a nucleic acid sequence having at least 90% sequence identity to SEQ ID NO: 41.
38. The method of embodiment 30 or the pharmaceutical composition of embodiment 30, wherein the genomic attachment site comprises a nucleic acid sequence having at least 90% sequence identity to SEQ ID NO: 60 and the vector attachment site comprises a nucleic acid sequence having at least 90% sequence identity to SEQ ID NO: 42.
39. The method of embodiment 30 or the pharmaceutical composition of embodiment 30, wherein the genomic attachment site comprises a nucleic acid sequence having at least 90% sequence identity to SEQ ID NO: 61 and the vector attachment site comprises a nucleic acid sequence having at least 90% sequence identity to SEQ ID NO: 43.
40. The method of embodiment 30 or the pharmaceutical composition of embodiment 30, wherein the genomic attachment site comprises a nucleic acid sequence having at least 90% sequence identity to SEQ ID NO: 62 and the vector attachment site comprises a nucleic acid sequence having at least 90% sequence identity to SEQ ID NO: 44.
41. The method of embodiment 30 or the pharmaceutical composition of embodiment 30, wherein the genomic attachment site comprises a nucleic acid sequence having at least 90% sequence identity to SEQ ID NO: 63 and the vector attachment site comprises a nucleic acid sequence having at least 90% sequence identity to SEQ ID NO: 45.
42. The method of embodiment 30 or the pharmaceutical composition of embodiment 30, wherein the genomic attachment site comprises a nucleic acid sequence having at least 90% sequence identity to SEQ ID NO: 64 and the vector attachment site comprises a nucleic acid sequence having at least 90% sequence identity to SEQ ID NO: 46.
43. The method of embodiment 30 or the pharmaceutical composition of embodiment 30, wherein the genomic attachment site comprises a nucleic acid sequence having at least 90% sequence identity to SEQ ID NO: 65 and the vector attachment site comprises a nucleic acid sequence having at least 90% sequence identity to SEQ ID NO: 47.
44. The method of embodiment 30 or the pharmaceutical composition of embodiment 30, wherein the genomic attachment site comprises a nucleic acid sequence having at least 90% sequence identity to SEQ ID NO: 66 and the vector attachment site comprises a nucleic acid sequence having at least 90% sequence identity to SEQ ID NO: 48.
45. The method of embodiment 30 or the pharmaceutical composition of embodiment 30, wherein the genomic attachment site comprises a nucleic acid sequence having at least 90% sequence identity to SEQ ID NO: 67 and the vector attachment site comprises a nucleic acid sequence having at least 90% sequence identity to SEQ ID NO: 49.
46. The method of embodiment 30 or the pharmaceutical composition of embodiment 30, wherein the genomic attachment site comprises a nucleic acid sequence having at least 90% sequence identity to SEQ ID NO: 68 and the vector attachment site comprises a nucleic acid sequence having at least 90% sequence identity to SEQ ID NO: 50.
47. The method of embodiment 28 or the pharmaceutical composition of embodiment 29, wherein the genomic attachment site comprises a nucleic acid sequence of SEQ ID NO: 51 and the vector attachment site comprises a nucleic acid sequence of SEQ ID NO: 31.
48. The method of embodiment 47 or the pharmaceutical composition of embodiment 47, wherein the genomic attachment site comprises a nucleic acid sequence of SEQ ID NO: 53 and the vector attachment site comprises a nucleic acid sequence of SEQ ID NO: 35.
49. The method of embodiment 47 or the pharmaceutical composition of embodiment 47, wherein the genomic attachment site comprises a nucleic acid sequence of SEQ ID NO: 54 and the vector attachment site comprises a nucleic acid sequence of SEQ ID NO: 36.
50. The method of embodiment 47 or the pharmaceutical composition of embodiment 47, wherein the genomic attachment site comprises a nucleic acid sequence of SEQ ID NO: 55 and the vector attachment site comprises a nucleic acid sequence of SEQ ID NO: 37.
51. The method of embodiment 47 or the pharmaceutical composition of embodiment 47, wherein the genomic attachment site comprises a nucleic acid sequence of SEQ ID NO: 56 and the vector attachment site comprises a nucleic acid sequence of SEQ ID NO: 38.
52. The method of embodiment 47 or the pharmaceutical composition of embodiment 47, wherein the genomic attachment site comprises a nucleic acid sequence of SEQ ID NO: 57 and the vector attachment site comprises a nucleic acid sequence of SEQ ID NO: 39.
53. The method of embodiment 47 or the pharmaceutical composition of embodiment 47, wherein the genomic attachment site comprises a nucleic acid sequence of SEQ ID NO: 58 and the vector attachment site comprises a nucleic acid sequence of SEQ ID NO: 40.
54. The method of embodiment 47 or the pharmaceutical composition of embodiment 47, wherein the genomic attachment site comprises a nucleic acid sequence of SEQ ID NO: 59 and the vector attachment site comprises a nucleic acid sequence of SEQ ID NO: 41.
55. The method of embodiment 47 or the pharmaceutical composition of embodiment 47, wherein the genomic attachment site comprises a nucleic acid sequence of SEQ ID NO: 60 and the vector attachment site comprises a nucleic acid sequence of SEQ ID NO: 42.
56. The method of embodiment 47 or the pharmaceutical composition of embodiment 47, wherein the genomic attachment site comprises a nucleic acid sequence of SEQ ID NO: 61 and the vector attachment site comprises a nucleic acid sequence of SEQ ID NO: 43.
57. The method of embodiment 47 or the pharmaceutical composition of embodiment 47, wherein the genomic attachment site comprises a nucleic acid sequence of SEQ ID NO: 62 and the vector attachment site comprises a nucleic acid sequence of SEQ ID NO: 44.
58. The method of embodiment 47 or the pharmaceutical composition of embodiment 47, wherein the genomic attachment site comprises a nucleic acid sequence of SEQ ID NO: 63 and the vector attachment site comprises a nucleic acid sequence of SEQ ID NO: 45.
59. The method of embodiment 47 or the pharmaceutical composition of embodiment 47, wherein the genomic attachment site comprises a nucleic acid sequence of SEQ ID NO: 64 and the vector attachment site comprises a nucleic acid sequence of SEQ ID NO: 46.
60. The method of embodiment 47 or the pharmaceutical composition of embodiment 47, wherein the genomic attachment site comprises a nucleic acid sequence of SEQ ID NO: 65 and the vector attachment site comprises a nucleic acid sequence of SEQ ID NO: 47.
61. The method of embodiment 47 or the pharmaceutical composition of embodiment 47, wherein the genomic attachment site comprises a nucleic acid sequence of SEQ ID NO: 66 and the vector attachment site comprises a nucleic acid sequence of SEQ ID NO: 48.
62. The method of embodiment 47 or the pharmaceutical composition of embodiment 47, wherein the genomic attachment site comprises a nucleic acid sequence of SEQ ID NO: 67 and the vector attachment site comprises a nucleic acid sequence of SEQ ID NO: 49.
63. The method of embodiment 47 or the pharmaceutical composition of embodiment 47, wherein the genomic attachment site comprises a nucleic acid sequence of SEQ ID NO: 68 and the vector attachment site comprises a nucleic acid sequence of SEQ ID NO: 50.
64. The method of embodiment 28 or the pharmaceutical composition of embodiment 29, wherein the genomic attachment site comprises a nucleic acid sequence having at least 90% sequence identity to SEQ ID NO: 31 and the vector attachment site comprises a nucleic acid sequence having at least 90% sequence identity to SEQ ID NO: 51.
65. The method of embodiment 64 or the pharmaceutical composition of embodiment 64, wherein the genomic attachment site comprises a nucleic acid sequence having at least 90% sequence identity to SEQ ID NO: 35 and the vector attachment site comprises a nucleic acid sequence having at least 90% sequence identity to SEQ ID NO: 53.
66. The method of embodiment 64 or the pharmaceutical composition of embodiment 64, wherein the genomic attachment site comprises a nucleic acid sequence having at least 90% sequence identity to SEQ ID NO: 36 and the vector attachment site comprises a nucleic acid sequence having at least 90% sequence identity to SEQ ID NO: 54.
67. The method of embodiment 64 or the pharmaceutical composition of embodiment 64, wherein the genomic attachment site comprises a nucleic acid sequence having at least 90% sequence identity to SEQ ID NO: 37 and the vector attachment site comprises a nucleic acid sequence having at least 90% sequence identity to SEQ ID NO: 55.
68. The method of embodiment 64 or the pharmaceutical composition of embodiment 64, wherein the genomic attachment site comprises a nucleic acid sequence having at least 90% sequence identity to SEQ ID NO: 38 and the vector attachment site comprises a nucleic acid sequence having at least 90% sequence identity to SEQ ID NO: 56.
69. The method of embodiment 64 or the pharmaceutical composition of embodiment 64, wherein the genomic attachment site comprises a nucleic acid sequence having at least 90% sequence identity to SEQ ID NO: 39 and the vector attachment site comprises a nucleic acid sequence having at least 90% sequence identity to SEQ ID NO: 57.
70. The method of embodiment 64 or the pharmaceutical composition of embodiment 64, wherein the genomic attachment site comprises a nucleic acid sequence having at least 90% sequence identity to SEQ ID NO: 40 and the vector attachment site comprises a nucleic acid sequence having at least 90% sequence identity to SEQ ID NO: 58.
71. The method of embodiment 64 or the pharmaceutical composition of embodiment 64, wherein the genomic attachment site comprises a nucleic acid sequence having at least 90% sequence identity to SEQ ID NO: 41 and the vector attachment site comprises a nucleic acid sequence having at least 90% sequence identity to SEQ ID NO: 59.
72. The method of embodiment 64 or the pharmaceutical composition of embodiment 64, wherein the genomic attachment site comprises a nucleic acid sequence having at least 90% sequence identity to SEQ ID NO: 42 and the vector attachment site comprises a nucleic acid sequence having at least 90% sequence identity to SEQ ID NO: 60.
73. The method of embodiment 64 or the pharmaceutical composition of embodiment 64, wherein the genomic attachment site comprises a nucleic acid sequence having at least 90% sequence identity to SEQ ID NO: 42 and the vector attachment site comprises a nucleic acid sequence having at least 90% sequence identity to SEQ ID NO: 61.
74. The method of embodiment 64 or the pharmaceutical composition of embodiment 64, wherein the genomic attachment site comprises a nucleic acid sequence having at least 90% sequence identity to SEQ ID NO: 44 and the vector attachment site comprises a nucleic acid sequence having at least 90% sequence identity to SEQ ID NO: 62.
75. The method of embodiment 64 or the pharmaceutical composition of embodiment 64, wherein the genomic attachment site comprises a nucleic acid sequence having at least 90% sequence identity to SEQ ID NO: 45 and the vector attachment site comprises a nucleic acid sequence having at least 90% sequence identity to SEQ ID NO: 63.
76. The method of embodiment 64 or the pharmaceutical composition of embodiment 64, wherein the genomic attachment site comprises a nucleic acid sequence having at least 90% sequence identity to SEQ ID NO: 46 and the vector attachment site comprises a nucleic acid sequence having at least 90% sequence identity to SEQ ID NO: 64.
77. The method of embodiment 64 or the pharmaceutical composition of embodiment 64, wherein the genomic attachment site comprises a nucleic acid sequence having at least 90% sequence identity to SEQ ID NO: 47 and the vector attachment site comprises a nucleic acid sequence having at least 90% sequence identity to SEQ ID NO: 65.
78. The method of embodiment 64 or the pharmaceutical composition of embodiment 64, wherein the genomic attachment site comprises a nucleic acid sequence having at least 90% sequence identity to SEQ ID NO: 48 and the vector attachment site comprises a nucleic acid sequence having at least 90% sequence identity to SEQ ID NO: 66.
79. The method of embodiment 64 or the pharmaceutical composition of embodiment 64, wherein the genomic attachment site comprises a nucleic acid sequence having at least 90% sequence identity to SEQ ID NO: 49 and the vector attachment site comprises a nucleic acid sequence having at least 90% sequence identity to SEQ ID NO: 67.
80. The method of embodiment 64 or the pharmaceutical composition of embodiment 64, wherein the genomic attachment site comprises a nucleic acid sequence having at least 90% sequence identity to SEQ ID NO: 50 and the vector attachment site comprises a nucleic acid sequence having at least 90% sequence identity to SEQ ID NO: 68.
81. The method of embodiment 28 or the pharmaceutical composition of embodiment 29, wherein the genomic attachment site comprises a nucleic acid sequence of SEQ ID NO: 31 and the vector attachment site comprises a nucleic acid sequence of SEQ ID NO: 51.
82. The method of embodiment 81 or the pharmaceutical composition of embodiment 81, wherein the genomic attachment site comprises a nucleic acid sequence of SEQ ID NO: 35 and the vector attachment site comprises a nucleic acid sequence of SEQ ID NO: 53.
83. The method of embodiment 81 or the pharmaceutical composition of embodiment 81, wherein the genomic attachment site comprises a nucleic acid sequence of SEQ ID NO: 36 and the vector attachment site comprises a nucleic acid sequence of SEQ ID NO: 54.
84. The method of embodiment 81 or the pharmaceutical composition of embodiment 81, wherein the genomic attachment site comprises a nucleic acid sequence of SEQ ID NO: 37 and the vector attachment site comprises a nucleic acid sequence of SEQ ID NO: 55.
85. The method of embodiment 81 or the pharmaceutical composition of embodiment 81, wherein the genomic attachment site comprises a nucleic acid sequence of SEQ ID NO: 38 and the vector attachment site comprises a nucleic acid sequence of SEQ ID NO: 56.
86. The method of embodiment 81 or the pharmaceutical composition of embodiment 81, wherein the genomic attachment site comprises a nucleic acid sequence of SEQ ID NO: 39 and the vector attachment site comprises a nucleic acid sequence of SEQ ID NO: 57.
87. The method of embodiment 81 or the pharmaceutical composition of embodiment 81, wherein the genomic attachment site comprises a nucleic acid sequence of SEQ ID NO: 40 and the vector attachment site comprises a nucleic acid sequence of SEQ ID NO: 58.
88. The method of embodiment 81 or the pharmaceutical composition of embodiment 81, wherein the genomic attachment site comprises a nucleic acid sequence of SEQ ID NO: 41 and the vector attachment site comprises a nucleic acid sequence of SEQ ID NO: 59.
89. The method of embodiment 81 or the pharmaceutical composition of embodiment 81, wherein the genomic attachment site comprises a nucleic acid sequence of SEQ ID NO: 42 and the vector attachment site comprises a nucleic acid sequence of SEQ ID NO: 60.
90. The method of embodiment 81 or the pharmaceutical composition of embodiment 81, wherein the genomic attachment site comprises a nucleic acid sequence of SEQ ID NO: 43 and the vector attachment site comprises a nucleic acid sequence of SEQ ID NO: 61.
91. The method of embodiment 81 or the pharmaceutical composition of embodiment 81, wherein the genomic attachment site comprises a nucleic acid sequence of SEQ ID NO: 44 and the vector attachment site comprises a nucleic acid sequence of SEQ ID NO: 62.
92. The method of embodiment 81 or the pharmaceutical composition of embodiment 81, wherein the genomic attachment site comprises a nucleic acid sequence of SEQ ID NO: 45 and the vector attachment site comprises a nucleic acid sequence of SEQ ID NO: 63.
93. The method of embodiment 81 or the pharmaceutical composition of embodiment 81, wherein the genomic attachment site comprises a nucleic acid sequence of SEQ ID NO: 46 and the vector attachment site comprises a nucleic acid sequence of SEQ ID NO: 64.
94. The method of embodiment 81 or the pharmaceutical composition of embodiment 81, wherein the genomic attachment site comprises a nucleic acid sequence of SEQ ID NO: 47 and the vector attachment site comprises a nucleic acid sequence of SEQ ID NO: 65.
95. The method of embodiment 81 or the pharmaceutical composition of embodiment 81, wherein the genomic attachment site comprises a nucleic acid sequence of SEQ ID NO: 48 and the vector attachment site comprises a nucleic acid sequence of SEQ ID NO: 66.
96. The method of embodiment 81 or the pharmaceutical composition of embodiment 81, wherein the genomic attachment site comprises a nucleic acid sequence of SEQ ID NO: 49 and the vector attachment site comprises a nucleic acid sequence of SEQ ID NO: 67.
97. The method of embodiment 81 or the pharmaceutical composition of embodiment 81, wherein the genomic attachment site comprises a nucleic acid sequence of SEQ ID NO: 50 and the vector attachment site comprises a nucleic acid sequence of SEQ ID NO: 68.
98. The method of any of embodiments 28-97, wherein the nucleic acid encoding a protein with serine integrase activity comprising an amino acid sequence having at least 80% sequence identity to SEQ ID NO: 1 is a nucleic acid according to any of embodiments 1-26 and wherein the protein with serine integrase activity comprising an amino acid sequence having at least 80% sequence identity to SEQ ID NO: 1 is a fusion protein according to embodiment 27.
99. The pharmaceutical composition of any of embodiments 29-97, wherein the nucleic acid encoding a protein with serine integrase activity comprising an amino acid sequence having at least 80% sequence identity to SEQ ID NO: 1 is a nucleic acid according to any of embodiments 1-26, wherein the protein with serine integrase activity comprising an amino acid sequence having at least 80% sequence identity to SEQ ID NO: 1 is a fusion protein according to claim 27 and wherein the cell is a cell comprising the nucleic acid of any of embodiments 1-26 and/or the fusion protein of embodiment 27, wherein the cell, optionally is a primary T cell.

Throughout the invention, the term "about" is intended to be understood as "+/- 10%". If "about" relates to a range, it refers to both lower and upper limits of the range. "A" is intended to mean "one or more", if not explicitly mentioned otherwise.

All literature cited herein is herewith fully incorporated. The present invention is further illustrated, but not limited, by the following examples.

### Brief description of the Drawings

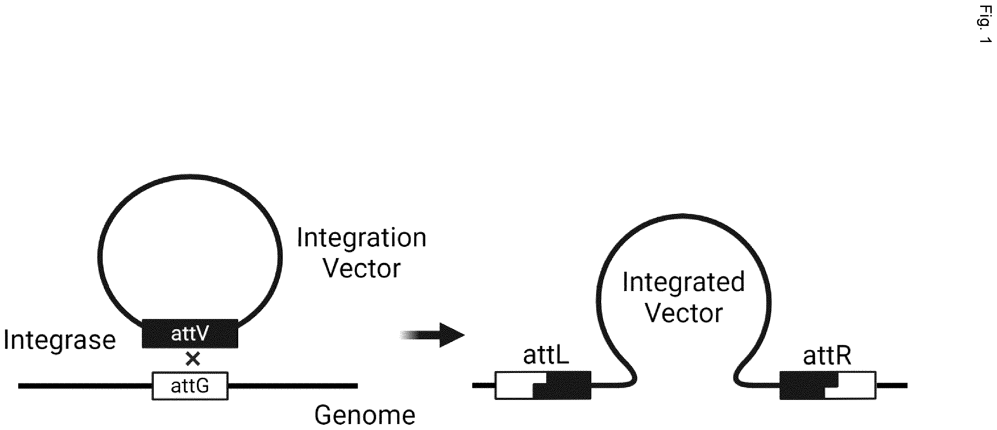

- **Figure 1:**: **Schematic overview of how serine integrases catalyze highly specific recombination between sequence-defined attachment (att) sites.** attG: attachment site in Genome | attV: attachment site on Integration Vector

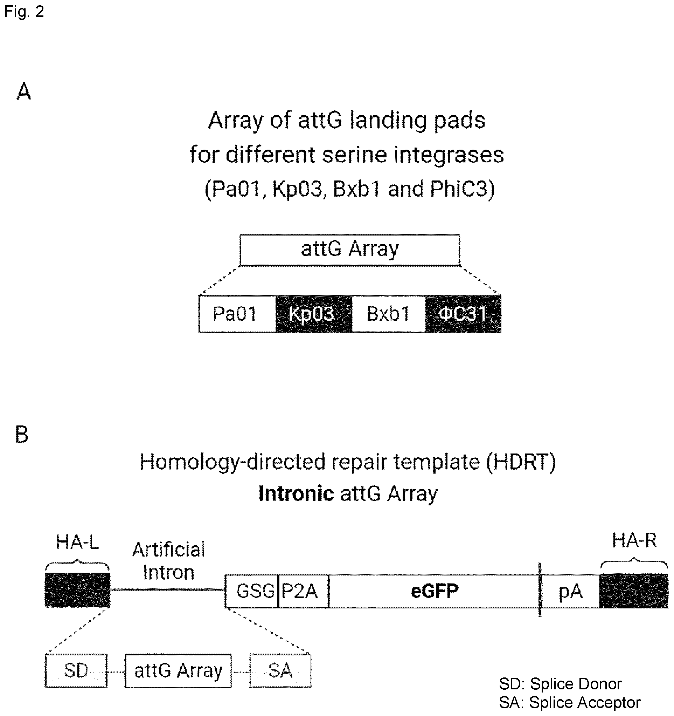

- **Figure 2:**: **Design of HDR templates for insertion into the GAPDH locus.** (A) Array of attG landing pads for the integrases Pa01, Kp03, Bxb1 and PhiC31. The sizes of the individual attG sites are listed on the right. (B) HDRT with the attG array in an artificial intron in front of the eGFP transgene. HA-UR: Homology-arm left/right; GSG: linker consisting of Gly-Ser-Gly; P2A: self-cleavage protein; pA: poly(A) sequence. (Figure created with BioRender.com)

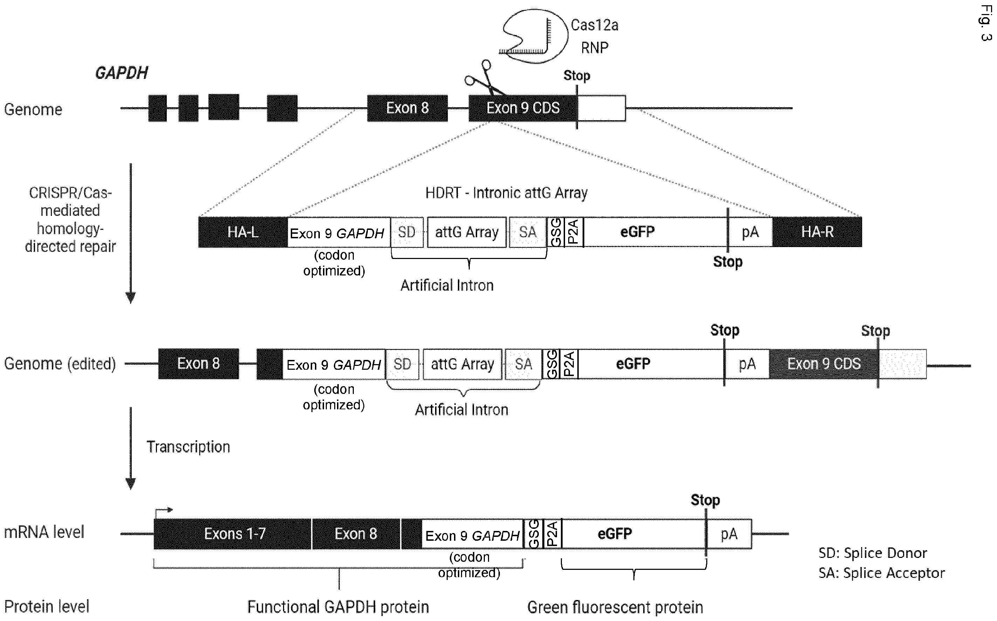

- **Figure 3:**: ***GAPDH* knock-in strategy on DNA, mRNA and protein level.** Schematic at molecular level showing the Cas12a knock-in strategy targeting the last exon of GAPDH. Restoration of recoded last exon 9 contained within the HDRT, alongside the intronic attG array and GFP reporter, is key for making a functional GAPDH protein. Cell survival is dependent on correct in-frame integration into the locus of this essential gene. Not drawn to scale. (Figure created with BioRender.com)

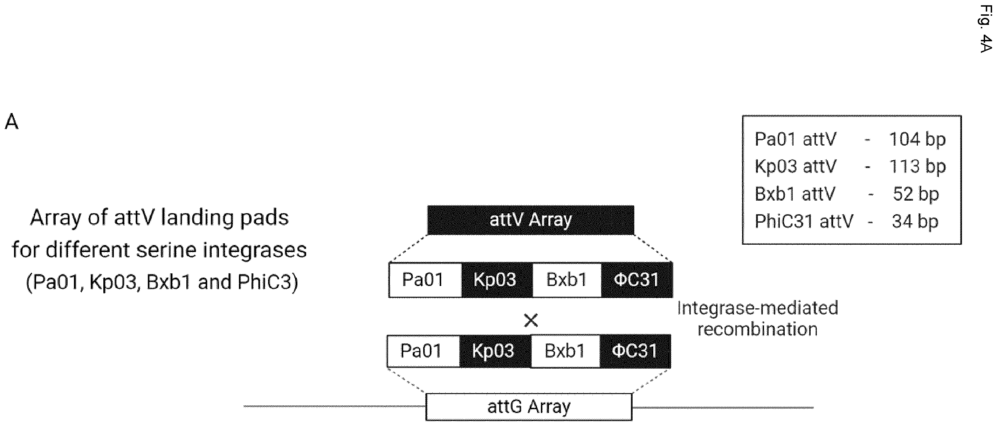

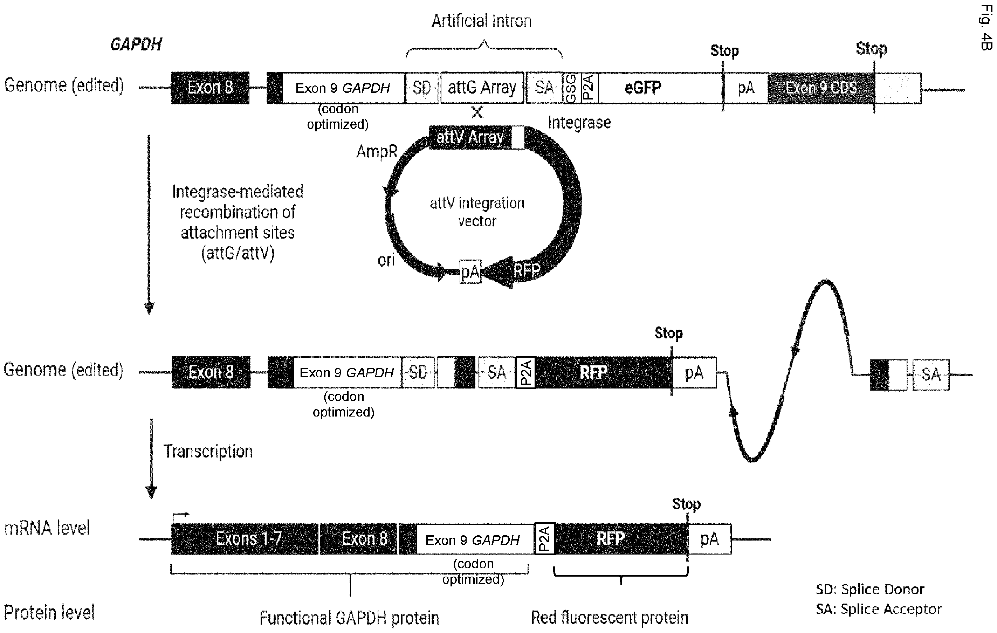

- **Figure 4:**: **Design of the attV integration vector.** (A) Arrray of attV landing pads for the intgerases Pa01, Kp03, Bxb1 and PhiC31. The size of the individual attV sequences is listed on the right. (B) Integrase-mediated site-specific insertion of the attV integration vector into the GAPDH locus on DNA, mRNA and protein level. Not drawn to scale. (Figure created with BioRender.com)

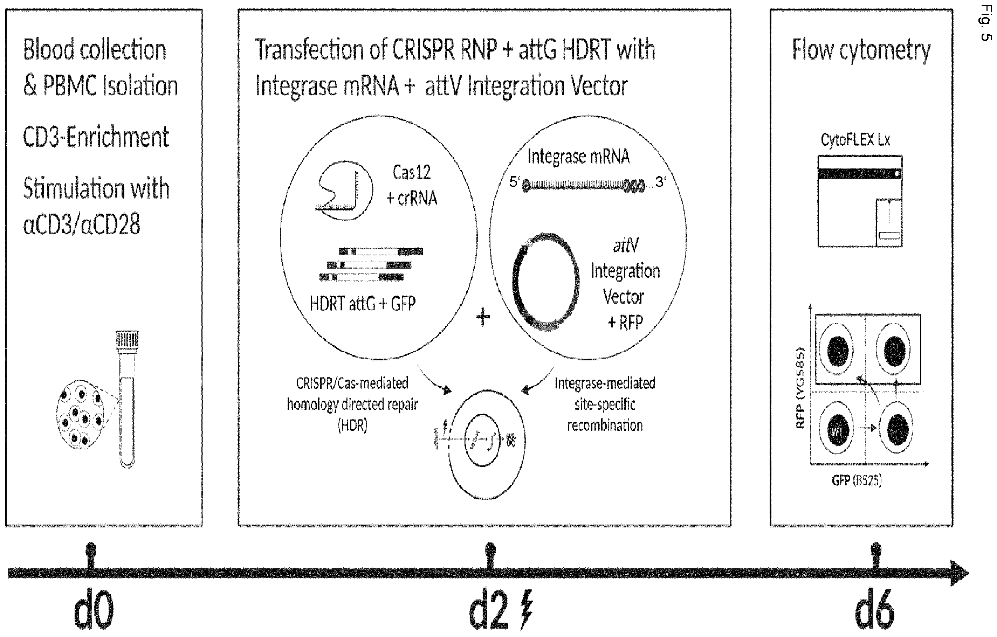

- **Figure 5:**: **Experimental workflow for single-step integration.** All components for successful vector integration, meaning Cas12a-RNP, attG HDRT, integrase mRNA and attV integration vector, are transfected in a single electroporation. (Figure created with BioRender.com)

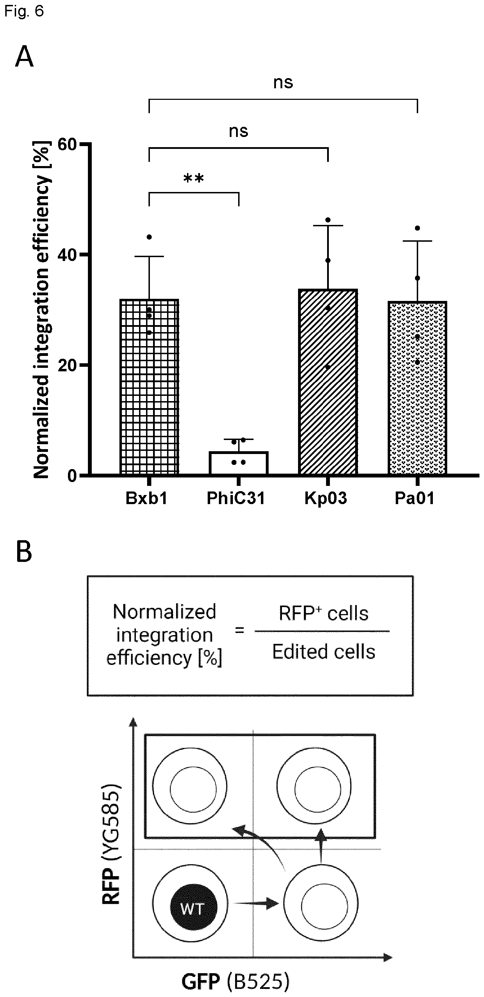

- **Figure 6:**: **Integration efficiencies of the tested serine integrases** - **normalized to edited, attG containing cells. (A, B)** Flow cytometry analysis of GFP and RFP on day 4 post-electroporation allowed the comparison of the tested serine integrases Pa01, Kp03, Bxb1 and PhiC31 by normalizing the percentage of RFP+ cells to total edited cell, comprising GFP⁺ and RFP⁺ cells. Statistical analysis of flow cytometry data from 4 donors was performed using a one-way ANOVA. Multiple comparisons were performed by comparing the mean of each column with the mean of every other column and corrected by the Turkey test. Asterisks represent different p-values calculated in the respective statistical tests (ns: p ≥ 0.05; **:p < 0.01).

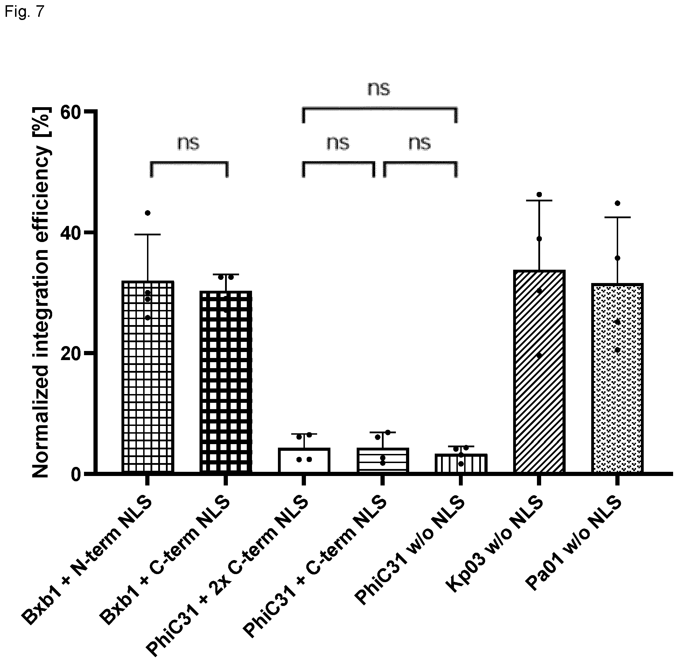

- **Figure 7:**: **Normalized integration efficiencies comparing different serine integrases with c- or n-terminal NLS against Pa01 and Kp03 without NLS.** Statistical analysis of flow cytometry data from 4 donors was performed using a one-way ANOVA. Multiple comparisons were performed by comparing the mean of each column with the mean of every other column and corrected by the Turkey test. Asterisks represent different p-values calculated in the respective statistical tests (ns: p ≥ 0.05; **:p < 0.01).

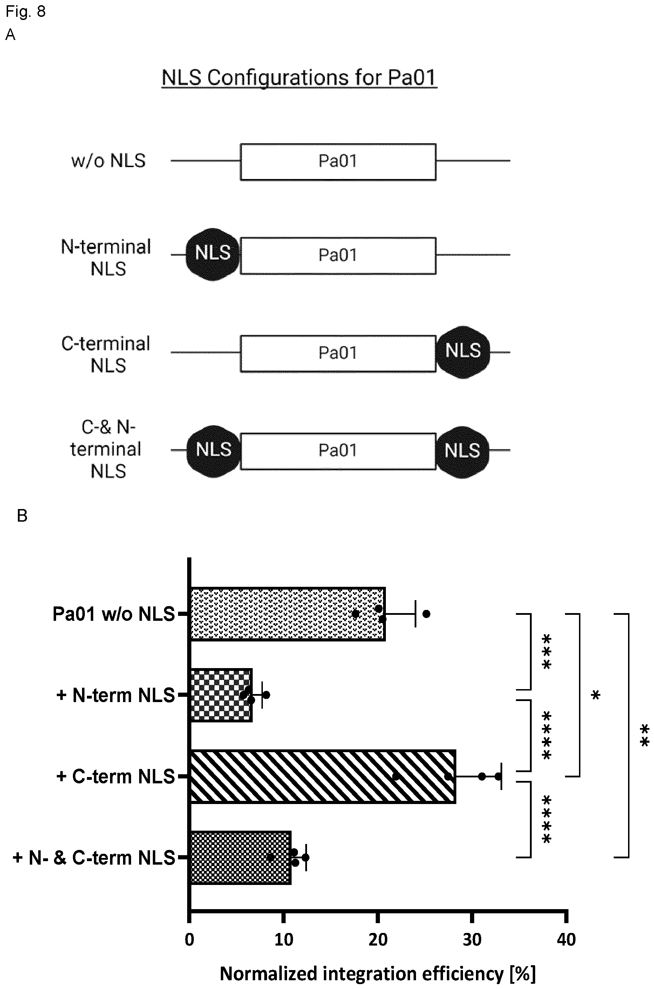

- **Figure 8:**: **Integration efficiencies of codon-optimized human Pa01 with various NLS configurations.** Pa01 without any NLS was modified by adding NLS N-terminally or C-terminally. Normalized integration efficiencies using these modified Pa01 integrases are shown on the right. Statistical analysis of flow cytometry data from 4 donors was performed using a one-way ANOVA. Multiple comparisons were performed by comparing the mean of each column with the mean of every other column and corrected by the Turkey test. Asterisks represent different p-values calculated in the respective statistical tests (ns: p _> 0.05; *: p < 0.05; **: p < 0.01; ***: p < 0.001; ****: p < 0.0001).

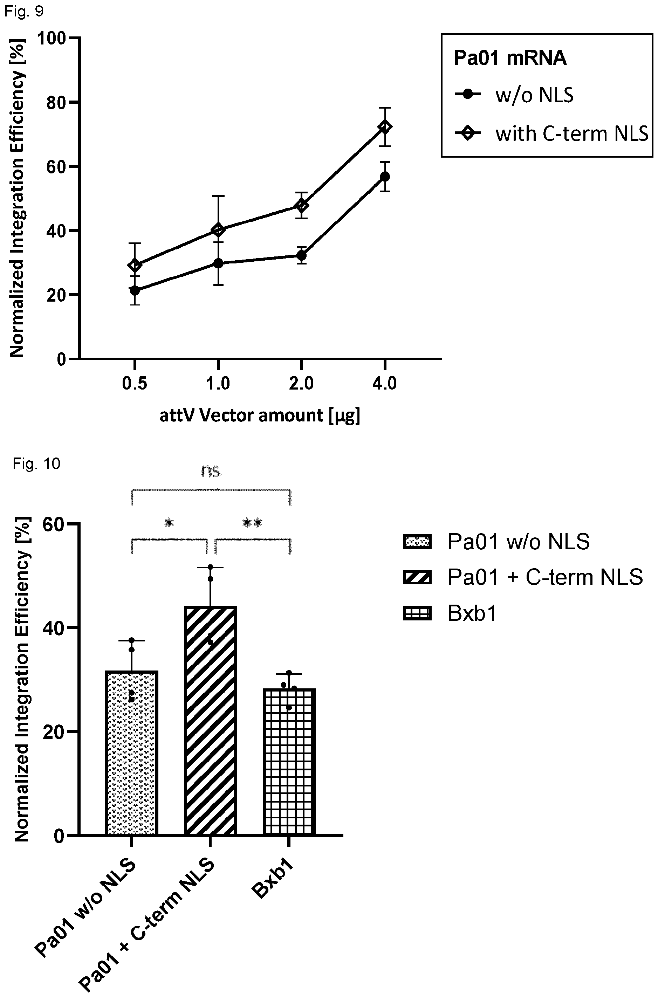

- **Figure 9:**: **Pa01 dose escalation experiment.** Stimulated T cells were transfected with increasing dosages of attV plasmid DNA vector with two different Pa01 versions - Pa01 without NLS and the optimized Pa01 with C-terminal NLS. The graphs show normalized integration efficiencies. (n = 5, healthy human donors)
- **Figure 10:**: **Paired comparison of normalized integration efficiencies of Pa01, optimized Pa01 and Bxb1.** Normalized integration efficiency of WT Pa01, optimized Pa01 (with C-terminal NLS) and Bxb1 for a dose level of 1 µg attV vector in paired experiments. Statistical analysis of flow cytometry data from 4 donors was performed using a one-way ANOVA of matched data. Multiple comparisons were performed by comparing the mean of each column with the mean of every other column and corrected by the Turkey test. Asterisks represent different p-values calculated in the respective statistical tests (ns: p ≥ 0.05; *: p < 0.05; **:p < 0.01).

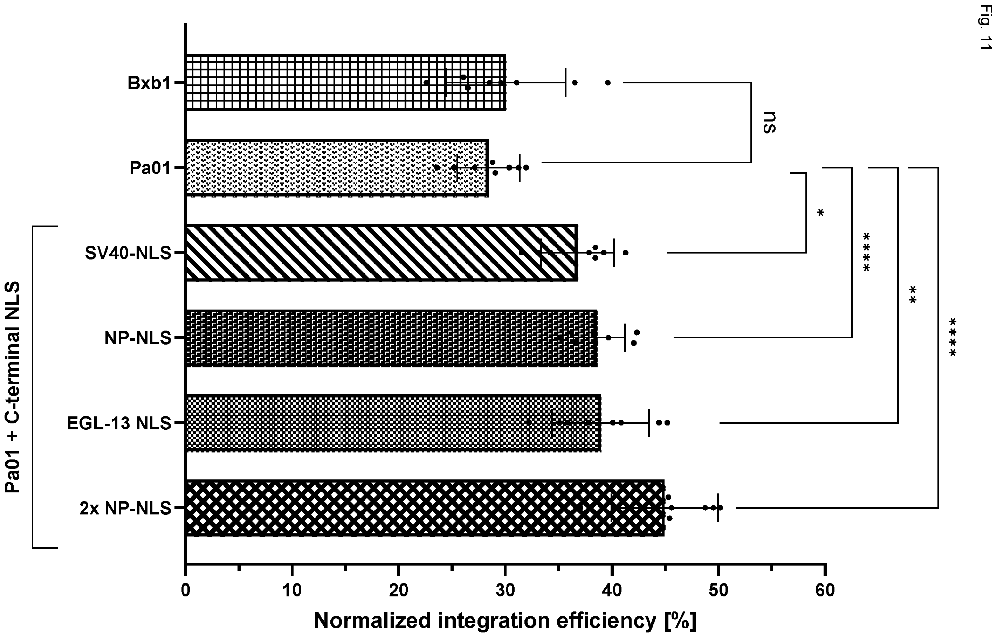

- **Figure 11:**: **Normalized integration efficiencies of Pa01 with different C-terminally fused NLS.** Codon-optimized human Pa01 was modified by the addition of different NLS at the C-terminus and the integration efficiencies of all Pa01-NLS variants were compared to unmodified Pa01 and the standard Bxb1 in paired experiments. Statistical analysis of flow cytometry data from 4 donors was performed using a one-way ANOVA of matched data with Geisser-Greenhouse correction. Multiple comparisons were performed by comparing the mean of each column with the mean of every other column and corrected by the Turkey test. Asterisks represent different p-values calculated in the respective statistical tests (ns: p ≥ 0.05; *:p < 0.05; **:p < 0.01; ***:p < 0.001; ****:p < 0.0001).

### Example:

### 1. Introduction

Immunotherapy with gene-edited T cells represents a potent approach for the treatment of various malignancies. The primary manufacturing method of engineered T cells, such as chimeric antigen receptor (CAR) T cells, relies on lentiviral transduction, which enables efficient introduction of DNA constructs into patient-derived T cells but entails significant costs and risks associated with semi-random integration of viral vectors. Alternatively, non-viral gene editing techniques are being explored, with current strategies using electroporation with programmable nucleases, such as CRISPR/Cas9, to induce homology-directed DNA repair (HDR) for targeted and precise insertion of DNA. However, these methods often suffer from low efficiency and limitations regarding DNA cargo size, thus compromising the therapeutic potential of engineered T cells.

Therefore, current gene-editing approaches face challenges in efficiently integrating large DNA constructs into specific genomic loci. To overcome this limitation, site-specific recombinases offer a promising strategy: Derived from bacteriophages, integrase systems have naturally evolved to catalyze DNA mobility to independently transfer foreign genetic material into bacterial host genomes without relying on endogenous genetic repair machinery (Durrant et al., 2023). As schematically depicted in **Fig. 1**, serine integrases mediate unidirectional recombination between specific attachment (att) sites, also known as landing pads, within both phage (attP) and bacterial (attB) genomes. Recombination of attP and attB site result in an integrated prophage flanked by two recombinant attachment sites, attL (left) and attR (right) (Stark, 2017)

Integrase-mediated DNA insertion through site-specific recombination can only be reversed in the presence of phage-encoded recombination directionality factors (RDFs), highlighting the site-selectivity of serine integrases for recombination (Merrick et al., 2018; Yan et al., 2013). Due to the absence of RDFs in human cells, integrated DNA cannot be excised through reversed recombination of the formed attL and attR sites, ensuring stable and precise DNA integration in various applications.

Leveraging the inherent ability of large serine integrases to mediate site-specific recombination between sequence-defined landing pads enables precise integration of sizeable DNA cargo at prespecified sites.

Large serine recombinases, such as Bxb1 and PhiC31, have already yielded useful genome engineering tools (Smith, 2015). PhiC31 has been exploited directly for genome targeting into pseudosite loci that resemble its native attachment sites (Growth et al., 2000; Keravala et al., 2006). Bxb1, on the other hand, has been used primarily as a landing pad integrase, where DNA cargos are integrated site-specifically into a pre-installed attachment site in a target genome (Inniss et al., 2017). A new RNA-guided Cas9-Bxb1 toolbox has recently expanded the precision of transgenesis and genetic engineering (Anzalone et al., 2022; Grandela et al., 2021; loannidi et al., 2021; Low et al., 2022). As large serine integrases naturally evolved to deliver long stretches of a viral genetic code, there is no reported upper limit on the size of the donor DNA, with reports demonstrating successful integration of up to 43 kb into mammalian cells (Hosur et al., 2022).

Multiple research groups have recently performed large-scale discovery of serine recombinases to identify novel serine recombinases suitable for genome engineering in mammalian cells (Durrant et al., 2023; Grandela et al., 2021). To date, only a limited number of these integrases have been evaluated for their efficacy in site-directed recombination within primary human T cells.

We developed a reporter-based screening system for evaluating serine integrase activity in gene editing applications targeting primary human T cells. The approach involves the combination of CRISPR/Cas-mediated HDR for targeted insertion of integrase attachment sites into the genome (attG) with integrase-mediated recombination of a DNA vector containing the respective attachment sites (attV). Four serine integrases (Bxb1, PhiC31, Kp03, and Pa01) were evaluated to identify the most promising candidate for gene editing in primary human T cells. The integration of larger multicistronic constructs through an integrase-based approach has the potential to open new possibilities for engineered T cells with enhanced functionalities.

### 2. Results

### 2.1 Array for Genomic Insertion of attG Landing Pads via HDR

For insertion of attG sites tagged with a green fluorescent protein (GFP) reporter transgene into the genome of primary human T cells via CRISPR/Cas-mediated HDR, we established a CRISPR/Cas12a knock-in strategy targeting exon 9 of the *GAPDH* gene, which was previously described to drive high transgene expression levels (Alllen et al., 2016; Kao et al., 2016).

The HDR template (HDRT) was designed to entail an array of attG landing pads encompassing attG sites specific to the integrases Pa01, Kp03, Bxb1 and PhiC31 alongside a GFP reporter transgene (Figure 2A). As in-frame insertion of the attG array into the *GAPDH* locus was not possible due to stop codons arising from the defined landing pad sequences, leading to disruption of downstream eGFP expression, the attG array was flanked by splice sites (a splice donor and acceptor), creating an artificial intron, that was placed upstream of the eGFP transgene (Figure 2B). This design of the HDRT facilitated simultaneous attG site insertion for all the integrases of interest in a single knock-in and ensured comparability among the integrases by establishing consistent conditions. Separate H DRTs for the different attG sites of the selected integrases could have resulted in varying knock-in efficiencies during separate transfections, with such initial discrepancies in knock-in efficiencies biasing the measured integration outcomes for the different integrases in subsequent analyses.

### 2.2 attV Integration Vector with RFP reporter for recombination into attG array

For serine integrase-mediated recombination into the attG array, we designed a plasmid DNA vector that included the corresponding attV landing pad array followed by a splice acceptor, a P2A self-cleavage peptide sequence, and a red fluorescent protein (RFP) reporter transgene, allowing RFP expression through the endogenous promoter upon in-frame integration of the vector into the genome (Figure 4B).

### 2.3 Single-step integration

Employing the described strategy, stimulated T cells were transfected with CRISPR RNP and HDRT for HDR-mediated attG landing pad insertion, while simultaneously co-delivering the serine integrase in the form of mRNA and the attV integration vector, all in one single electroporation (Figure 5). Delivery of the serine integrase in the form of mRNA or protein - rather than encoded in plasmid DNA - mitigates toxicity and is less immunogenic.

### 2.4 Comparing the serine integrases Bxb1, PhiC31, Kp03 and Pa01

Using the integrase screening system combining CRISPR/Cas-mediated HDR for GFP-tagged attG landing pad array insertion and RFP-tagged integrase-mediated site-specific attV vector recombination in a single-step approach, we compared the efficacy of four distinct serine integrases: Bxb1, PhiC31, Kp03 and Pa01. Integrase mRNA was generated through *in-vitro* transcription (IVT) from plasmids containing the respective integrase sequences. The mRNA was used to transfect stimulated primary T cells alongside CRISPR RNP, attG HDRT and attV vector. The integration efficiency of these various integrases was subsequently assessed by flow cytometry analysis of RFP and GFP expression within the cells. The percentage of RFP+ cells was normalized to the total edited cells (comprising both GFP+ and RFP+ cells), compensating for the variable knock-in efficiencies of the attG landing pad array induced by CRISPR/Cas-mediated HDR in independent donors and experiments. The integrase Pa01 demonstrated normalized integration efficiencies comparable to Kp03 and the well-established Bxb1, averaging around 30% (Figure 6). In contrast, PhiC31 exhibited significantly lower efficiency under the same conditions.

### 2.5 Optimization of Pa01 by addition of a C-terminal nuclear localization sequence (NLS)

The established serine integrase Bxb1 has previously been modified with an N-terminal SV40 nuclear localization sequence (NLS) to improve its activity. NLS are peptide sequences that facilitate the transport of proteins into the nucleus through nuclear pore complexes. These signals are recognized by importin proteins, which mediate the transport of cargo proteins across the nuclear envelope. For Bxb1, it was recently described that not only N-terminal fusion but also C-terminal fusion of NLS both enhance its integration efficiency in CHO cells - an immortalized epithelial cell line derived from hamster ovary -, while Bxb1 with a C-terminal NLS demonstrated the highest enhancement in integration efficiency among tested NLS fusions (Huhtinen et al., 2024). Notably, in this study, the Bxb1 variants were not only used in non-human cells, but also delivered encoded in plasmid DNA, deviating from our approach of mRNA or protein delivery. Based on these findings, we tested mRNA delivery of Bxb1 with a C-terminal NLS against the previously used Bxb1 with N-terminal fusion in primary human cells. Comparing normalized integration efficiencies of both Bxb1 variants demonstrated comparable efficiencies and no significant improvement with the C-terminal NLS (**Fig. 7**).

We also tested a previously used version of PhiC31 with two NLS fused to the C-terminus against a variant featuring only one C-terminal NLS and unmodified PhiC31. Despite previous descriptions of beneficial C-terminal NLS on PhiC31, analyzing the integration efficiencies in primary human cells did not a reveal a significant improvement of the integrase's activity with C-terminal fusions of NLS compared to the unmodified PhiC31 (**Fig. 7**).

As Pa01 showed integration efficiencies comparable to the established Bxb1 integrase and was reported to have only minimal off-target effects - in contrast to Kp03, which was excluded from further study due to significant off-target activity in mammalian cells (Durrant et al., 2023) - we investigated how the addition of an NLS to Pa01 would impact integration and whether Pa01 could be optimized for enhanced activity.

Multiple constructs were cloned encompassing the human codon-optimized Pa01 sequence with distinct NLS configurations - C-terminal, N-terminal, and on both ends (Fehler! Verweisquelle konnte nicht gefunden werden.). Following generation of the mRNA through in-vitro transcription (IVT), T cells were transfected with each Pa01 variant mRNA featuring the various NLS configurations within the single-step integration approach.

Comparing the normalized integrations efficiencies of the Pa01 variants encompassing NLS modifications to the unmodified Pa01, revealed that the addition of a C-terminal NLS significantly increased Pa01's activity. Surprisingly, adding an N-terminal NLS impaired Pa01's activity, reducing the integration efficiency more than two-fold (**Fig. 8B**). The diminished integration efficiency observed with the N-terminal modification of Pa01 was not rescued by the supplementary addition of a C-terminal NLS. These results differ from previous experiments with Bxb1, which performs similarly well in primary human cells, regardless of whether the NLS is positioned at the c- or n-terminus (**Fig. 7**). Further, the advantageous effect of the c-terminal modification was not obvious, because another PhiC31 integrase did not benefit from c-terminal NLS, when delivered via mRNA or protein into primary human cells (**Fig. 7**).

### 2.6 Dose Escalation Experiments for Pa01 variants with and without C-terminal NLS

To compare the integration efficiency of the Pa01 variant with C-terminal NLS and unmodified Pa01 across various dosages of attV vector, a dose-escalation experiment was conducted with both Pa01 variants, in which isolated T cells were transfected with increasing amounts of attV plasmid. The results revealed a distinct dose-dependency in Pa01's integration efficiency concerning the attV integration vector (**Fig. 9**). Notably, Pa01 with a C-terminal NLS consistently outperformed the unmodified Pa01 in all conditions, starting with normalized integration efficiencies of 30% for the lowest amount of transfected attV plasmid (0.5 µg) reaching up to 75% for the highest transfected amount (4 µg).

### 2.7 Pa01-NLS exhibits enhanced efficiency compared to WT Pa01 and Bxb1

The results from these previous experiments suggested enhanced activity of Pa01 with incorporation of a C-terminal NLS. To further investigate how this optimized Pa01 variant would compare to the established Bxb1 integrase (Anzalone et al., 2022), the optimized Pa01 was tested against Bxb1 and the unmodified Pa01 without NLS in paired experiments for an attV vector dose-level of 1 µg. The unmodified Pa01 and Bxb1 - both showing comparable integration efficiencies of around 30% - were consistently outperformed by the optimized Pa01 with C-terminal NLS in all paired experiments (Fehler! Verweisquelle konnte nicht gefunden werden. **10**). The optimized Pa01 facilitated recombination of the attV vector in more than 40% of attG landing pad containing cells on average. Therefore, Pa01 with c-terminal NLS represents a superior integrase over state of the art.

### 2.8 Integration efficiencies of Pa01 with different C-terminally fused NLS

We hypothesized that the improved integration efficiency observed for Pa01 with a C-terminal NLS in human cells is not limited to the use of a specific NLS (i.e., an SV40 NLS), but could also be achieved by fusing alternative NLS, including different types of NLS such as "classical" monopartite or bipartite NLS as well as "non-classical" NLS, to the C-terminus of Pa01. To this end, we therefore constructed additional Pa01 variants containing either a nucleoplasmin (NP) NLS (SEQ ID NO: 5), an EGL-13 NLS (SEQ ID NO: 14) or an NLS comprising two copies of the NP NLS sequence (SEQ ID NO: 15) at the C-terminus. Compared to unmodified Pa01, all tested Pa01 variants with C-terminal NLS exhibited significantly increased integration rates **(****Fig. 11****),** thus confirming our hypothesis.

### References

Micklethwaite KP, Gowrishankar K, Gloss BS, Li Z, Street JA, Moezzi L, Mach MA, Sutrave G, Clancy LE, Bishop DC, Louie RHY, Cai C, Foox J, MacKay M, Sedlazeck FJ, Blombery P, Mason CE, Luciani F, Gottlieb DJ, Blyth E. Investigation of product-derived lymphoma following infusion of piggyBac-modified CD19 chimeric antigen receptor T cells. Blood. 2021 Oct 21;138(16):1391-1405. doi: 10.1182/blood.2021010858. PMID: 33974080; PMCID: PMC8532197.

Durrant MG, Fanton A, Tycko J, Hinks M, Chandrasekaran SS, Perry NT, Schaepe J, Du PP, Lotfy P, Bassik MC, Bintu L, Bhatt AS, Hsu PD. Systematic discovery of recombinases for efficient integration of large DNA sequences into the human genome. Nat Biotechnol. 2023 Apr;41(4):488-499. doi: 10.1038/s41587-022-01494-w. Epub 2022 Oct 10. PMID: 36217031; PMCID: PMC10083194.

Campbell AM (1962) Episomes. In Advances in Genetics, Caspari EW, Thoday JM (eds) pp 101-145. New York, NY: Academic Press.

Stark WM. Making serine integrases work for us. Curr Opin Microbiol. 2017 Aug;38:130-136. doi: 10.1016/j.mib.2017.04.006. Epub 2017 Jun 6. PMID: 28599144.

Lu, J., Wu, T., Zhang, B. et al. Types of nuclear localization signals and mechanisms of protein import into the nucleus. Cell Commun Signal 19, 60 (2021). https://doi.org/10.1186/s12964-021-00741-y

Hosur, V., Low, B. E. & Wiles, M. V. Programmable RNA-Guided Large DNA Transgenesis by CRISPR/Cas9 and Site-Specific Integrase Bxb1. Front. Bioeng. Biotechnol. 10, 910151 (2022). https://en.wikipedia.org/wiki/Prime_editing

Anzalone AV, Gao XD, Podracky CJ, Nelson AT, Koblan LW, Raguram A, Levy JM, Mercer JAM, Liu DR. Programmable deletion, replacement, integration and inversion of large DNA sequences with twin prime editing. Nat Biotechnol. 2022 May;40(5):731-740. doi: 10.1038/s41587-021-01133-w. Epub 2021 Dec 9. PMID: 34887556; PMCID: PMC9117393.

Yarnall MTN, loannidi El, Schmitt-Ulms C, Krajeski RN, Lim J, Villiger L, Zhou W, Jiang K, Garushyants SK, Roberts N, Zhang L, Vakulskas CA, Walker JA 2nd, Kadina AP, Zepeda AE, Holden K, Ma H, Xie J, Gao G, Foquet L, Bial G, Donnelly SK, Miyata Y, Radiloff DR, Henderson JM, Ujita A, Abudayyeh OO, Gootenberg JS. Drag-and-drop genome insertion of large sequences without double-strand DNA cleavage using CRISPR-directed integrases. Nat Biotechnol. 2023 Apr;41(4):500-512. doi: 10.1038/s41587-022-01527-4. Epub 2022 Nov 24. PMID: 36424489; PMCID: PMC10257351.

Merrick, C. A., Zhao, J. & Rosser, S. J. Serine Integrases: Advancing Synthetic Biology. ACS Synth. Biol. 7, 299-310 (2018).

Yan, B.-W., Zhao, Y.-F., Cao, W.-G., Li, N. & Gou, K.-M. Mechanism of random integration of foreign DNA in transgenic mice. Transgenic Res. 22, 983-992 (2013).

Smith, M. C. M. Phage-encoded Serine Integrases and Other Large Serine Recombinases. Microbiol. Spectr. 3, 10.1128/microbiolspec.mdna3-0059-2014 (2015).

Groth, A. C., Olivares, E. C., Thyagarajan, B. & Calos, M. P. A phage integrase directs efficient site-specific integration in human cells. Proc. Natl. Acad. Sci. U. S. A. 97, 5995-6000 (2000). Keravala, A. et al. A diversity of serine phage integrases mediate site-specific recombination in mammalian cells. Mol. Genet. Genomics MGG 276, 135-146 (2006).

Inniss, M. C. et al. A novel Bxb1 integrase RMCE system for high fidelity site-specific integration of mAb expression cassette in CHO Cells. Biotechnol. Bioeng. 114, 1837-1846 (2017). Grandela, C. et al. STRAIGHT-IN: A platform for high-throughput targeting of large DNA payloads into human pluripotent stem cells. 2021.12.08.471715 Preprint at https://doi.org/10.1101/2021.12.08.471715 (2021).

loannidi, E. I. et al. Drag-and-drop genome insertion without DNA cleavage with CRISPR-directed integrases. 2021.11.01.466786 Preprint at https://doi.org/10.1101/2021.11.01.466786 (2021). Low, B. E., Hosur, V., Lesbirel, S. & Wiles, M. V. Efficient targeted transgenesis of large donor DNA into multiple mouse genetic backgrounds using bacteriophage Bxb1 integrase. Sci. Rep. 12, 5424 (2022).

Hosur, V., Low, B. E. & Wiles, M. V. Programmable RNA-Guided Large DNA Transgenesis by CRISPR/Cas9 and Site-Specific Integrase Bxb1. Front. Bioeng. Biotechnol. 10, 910151 (2022). Allen, A. G. et al. A highly efficient transgene knock-in technology in clinically relevant cell types. Nat. Biotechnol. 1-12 (2023) doi:10.1038/s41587-023-01779-8.

Kao, T. et al. GAPTrap: A Simple Expression System for Pluripotent Stem Cells and Their Derivatives. Stem Cell Rep. 7, 518-526 (2016).

Huhtinen, O., Prince, S., Lamminmäki, U., Salbo, R. & Kulmala, A. Increased stable integration efficiency in CHO cells through enhanced nuclear localization of Bxb1 serine integrase. BMC Biotechnol. 24, 44 (2024).

Inniss MC, Bandara K, Jusiak B, Lu TK, Weiss R, Wroblewska L, Zhang L. A novel Bxb1 integrase RMCE system for high fidelity site-specific integration of mAb expression cassette in CHO Cells. Biotechnol Bioeng. 2017 Aug;114(8):1837-1846. doi: 10.1002/bit.26268. Epub 2017 Mar 14. PMID: 28186334.

Colloms SD, Merrick CA, Olorunniji FJ, Stark WM, Smith MC, Osbourn A, Keasling JD, Rosser SJ. Rapid metabolic pathway assembly and modification using serine integrase site-specific recombination. Nucleic Acids Res. 2014 Feb;42(4):e23. doi: 10.1093/nar/gkt1101. Epub 2013 Nov 12. PMID: 24225316; PMCID: PMC3936721.

Fauser et al., 2004, Systematic Development of Reprogrammed Modular Integrases Enables Precise Genomic Integration of Large DNA Sequences. bioRxiv. https://doi.org/10.1101/2024.05.09.593242.

Durrant MG, Perry NT, Pai JJ, Jangid AR, Athukoralage JS, Hiraizumi M, McSpedon JP, Pawluk A, Nishimasu H, Konermann S, Hsu PD. Bridge RNAs direct programmable recombination of target and donor DNA. Nature. 2024 Jun;630(8018):984-993. doi: 10.1038/s41586-024-07552-4. Epub 2024 Jun 26. PMID: 38926615; PMCID: PMC11208160.

Ferreira da Silva J, Tou CJ, King EM, Eller ML, Rufino-Ramos D, Ma L, Cromwell CR, Metovic J, Benning FMC, Chao LH, Eichler FS, Kleinstiver BP. Click editing enables programmable genome writing using DNA polymerases and HUH endonucleases. Nat Biotechnol. 2024 Jul 22. doi: 10.1038/s41587-024-02324-x. Epub ahead of print. PMID: 39039307.

Liu B, Dong X, Zheng C, Keener D, Chen Z, Cheng H, Watts JK, Xue W, Sontheimer EJ. Targeted genome editing with a DNA-dependent DNA polymerase and exogenous DNA-containing templates. Nat Biotechnol. 2024 Jul;42(7):1039-1045. doi: 10.1038/s41587-023-01947-w. Epub 2023 Sep 14. PMID: 37709915. https://en.wikipedia.org/wiki/CRISPR-associated_transposons

## Claims

1. A nucleic acid encoding a fusion protein comprising
a) a serine integrase consisting of a protein having at least 80% sequence identity to SEQ ID NO: 1 and
b) a nuclear localization signal (NLS) at the C-terminus of the serine integrase.

2. The nucleic acid of claim 1, wherein the NLS comprises
a) at least one NLS comprising a consensus sequence of KX₁X₂X₃, wherein X₁ and X₃ can be either K or R and X₂ can be any amino acid, and/or
b) at least one NLS comprising a consensus sequence of X₁(X₂)₁₀₋₁₂KRX₃K, where X₁ can be either K or R and X₂ and X₃ can be any amino acid, and/or
c) at least one NLS selected from the group comprising SEQ ID NO: 10-14.

3. The nucleic acid of any of the preceding claims, wherein the NLS comprises any of SEQ ID NO: 2; SEQ ID NO: 3, SEQ ID NO: 4; SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15 or any combination thereof.

4. The nucleic acid of any of the preceding claims, wherein the fusion protein comprises another segment at the C-terminus or between the serine integrase and the NLS.

5. The nucleic acid of any of the preceding claims, wherein the nucleic acid is RNA, preferably mRNA.

6. The nucleic acid of any of claims 1-4, wherein the nucleic acid is DNA.

7. A fusion protein comprising a serine integrase encoded by the nucleic acid of any of the preceding claims.

8. Use of the nucleic acid of any of claims 1-6 or of the fusion protein of claim 7 for preparing an engineered cell comprising a cargo sequence, optionally, *in vitro* or ex *vivo.*

9. A method of preparing an engineered cell comprising a cargo sequence in its genomic DNA, comprising steps of:
a. optionally, site-specifically inserting one or more genomic attachment sites (attG sites) comprising a nucleic acid sequence having at least 90% sequence identity to any of SEQ ID NO: 31 or SEQ ID NO: 51 into the genomic DNA of a cell, and
b. contacting the cell with a nucleic acid encoding a protein with serine integrase activity comprising an amino acid sequence having at least 80% sequence identity to SEQ ID NO: 1 or with a protein with serine integrase activity comprising an amino acid sequence having at least 80% sequence identity to SEQ ID NO: 1 and with a DNA comprising i) one or more vector attachment sites (attV sites) comprising a nucleic acid sequence having at least 90% sequence identity to any of SEQ ID NO: 31 or SEQ ID NO: 51 and ii) the cargo sequence,
wherein the one or more genomic attachment sites and the one or more vector attachment sites correspond to each other to enable serine integrase-mediated recombination and wherein the method can be an *in vivo, ex vivo* or *in vitro* method.

10. The method of claim 9, wherein the nucleic acid encoding a protein with serine integrase activity comprising an amino acid sequence having at least 80% sequence identity to SEQ ID NO: 1 is a nucleic acid according to any of claims 1-6 and wherein the protein with serine integrase activity comprising an amino acid sequence having at least 80% sequence identity to SEQ ID NO: 1 is a fusion protein according to claim 7.

11. A cell comprising the nucleic acid of any of claims 1-6 and/or the fusion protein of claim 7, wherein the cell, optionally is a primary T cell.

12. A pharmaceutical composition comprising
a) a nucleic acid encoding a protein with serine integrase activity comprising an amino acid sequence having at least 80% sequence identity to SEQ ID NO: 1 or a protein with serine integrase activity comprising an amino acid sequence having at least 80% sequence identity to SEQ ID NO: 1, or a cell comprising said nucleic acid and/or said protein,
b) a DNA comprising at least one vector attachment site comprising or consisting of a nucleic acid sequence having at least 90% sequence identity to any of SEQ ID NO: 31 or 51 and a cargo sequence,
c) a source for a gene editor capable of site-specifically introducing at least one genomic attachment site into the genomic DNA of a cell,
d) a nucleic acid providing at least one genomic attachment site comprising or consisting of a nucleic acid sequence having at least 90% sequence identity to any of SEQ ID NO: 31 or 51 that corresponds to the at least one vector attachment site in the DNA according to b) to enable serine integrase-mediated recombination,
e) optionally, at least one carrier and/or excipient.

13. The pharmaceutical composition of claim 12, wherein the nucleic acid encoding a protein with serine integrase activity comprising an amino acid sequence having at least 80% sequence identity to SEQ ID NO: 1 is a nucleic acid according to any of claims 1-6, wherein the protein with serine integrase activity comprising an amino acid sequence having at least 80% sequence identity to SEQ ID NO: 1 is a fusion protein according to claim 7 and wherein the cell is a cell of claim 11.

14. The pharmaceutical composition of any of claims 12 or 13 comprising the nucleic acid and/or protein for use in *in vivo* gene therapy.

15. The pharmaceutical composition of any of claims 12 or 13 comprising the cell for ex *vivo* gene therapy.

16. The pharmaceutical composition of any of claims 12-13 for use in treating a disease or disorder selected from the group comprising cancer, a chronic inflammatory disorder, an autoimmune disorder, a musculoskeletal disorder, a cardiovascular disorder, a hereditary disease, a metabolic disease, a degenerative disorder, a neurological disorder, a sensory disorder, and an infection, optionally, for use in adoptive T cell therapy.
